# EUROPEAN PATENT SPECIFICATION

(11) **EP 2 582 384 B1**
(45) Date of publication and mention of the grant of the patent: **23.11.2016**
(21) Application number: 11796346.2
(22) Date of filing: 15.06.2011
(51) Int. Cl.: A61K 38/17, A61K 38/10, A61K 31/711, A61K 31/70, A61P 35/00, A61K 31/7105

(54) **COMPOSITIONS AND METHODS FOR TREATING CANCER**
ZUSAMMENSETZUNGEN UND VERFAHREN ZUR BEHANDLUNG VON KREBS
COMPOSITIONS ET PROCÉDÉS DE TRAITEMENT DU CANCER

(30) Priority: 25.01.2011 US 201161436030 P; 15.06.2010 US 355134 P
(43) Date of publication of application: 24.04.2013
(73) Proprietor: THE CLEVELAND CLINIC FOUNDATION, Cleveland, OH 44195 (US)
(72) Inventor: HAZEN, Stanley L., Pepper Pike, Ohio 44124 (US); DIDONATO, Joseph, Westlake, Ohio 44145 (US); ZAMANIAN-DARYOUSH, Maryam, Shaker Heights, Ohio 44120 (US)
(74) Representative: Glawe, Delfs, Moll
(86) International application number: PCT/US2011/040471
(87) International publication number: WO 2011/159771

(56) References cited:
- WO-A1-2009/055538
- CA-A1- 2 727 811
- US-A- 5 192 264
- US-A1- 2006 172 429
- US-A1- 2006 172 919
- HAN CUNZHI ET AL: "Serum levels of leptin, insulin, and lipids in relation to breast cancer in China", ENDOCRINE, HUMANA PRESS, US, vol. 26, no. 1, 1 February 2005 (2005-02-01), pages 19-24, XP009175427, ISSN: 0969-711X
- H. DUEZ: "Regulation of Human ApoA-I by Gemfibrozil and Fenofibrate Through Selective Peroxisome Proliferator-Activated Receptor Modulation", ARTERIOSCLEROSIS, THROMBOSIS, AND VASCULAR BIOLOGY, vol. 25, no. 3, 1 March 2005 (2005-03-01), pages 585-591, XP055095620, ISSN: 1079-5642, DOI: 10.1161/01.ATV.0000154140.73570.00
- RENANA SHOR ET AL.: 'Low HDL levels and the risk of death, sepsis and malignancy' CLIN. RES. CARDIOL. vol. 97, no. 4, 2008, pages 227 - 233, XP019589025
- MASASHI UEDA ET AL.: 'Establishment and evaluation of 2 monoclonal antibodies against oxidized apolipoprotein A-I (apoA-I) and its application to determine blood oxidized apoA-I levels' CLINICA CHIMICA ACTA vol. 378, no. 1/2, 2007, pages 105 - 111, XP005891495
- DOROTHEE WEIHRAUCH ET AL.: 'Effects of D-4F on vasodilation, oxidative stress, angiostatin, myocardial inflammation, and angiogenic potential in tight-skin mice' AM. J. PHYSIOL. HEART CIRC. PHYSIOL. vol. 293, no. 3, 2007, pages H1432 - H1441, XP055073117

## Description

### FIELD OF THE INVENTION

The present invention relates to methods for treating cancer in a subject, as well as related compositions and kits, that employ a therapeutic agent, or a nucleic acid sequence encoding a therapeutic agent, selected from apolipoprotein A-1 (ApoA1), an ApoA1 mimetic, an agent that increases expression of ApoA1, or a binding agent specific for oxidized ApoA1. In certain examples, the ApoA1 or ApoA1 mimetic is at least partially oxidation resistant.

### BACKGROUND OF THE INVENTION

Circulating cholesterol is carried by plasma lipoproteins. Lipoproteins are particles of lipid and protein that transport lipids in the blood. Low-density lipoproteins (LDL) and high-density lipoproteins (HDL) are the major cholesterol carriers. LDL is believed to be responsible for the delivery of cholesterol from the liver to extrahepatic tissues in the body.

The term "reverse cholesterol transport" (RCT) describes the transport of cholesterol from extrahepatic tissues to the liver where it is catabolized and eliminated. It is believed that plasma HDL particles play a major role in the reverse transport process, acting as scavengers of tissue cholesterol. RCT consists mainly of three steps: (a) cholesterol efflux, the initial removal of cholesterol from various pools of peripheral cells; (b) cholesterol esterification by the action of lecithin:cholesterol acyltransferase (LCAT), preventing a re-entry of effluxed cholesterol into cells; and (c) uptake/delivery of HDL cholesterol ester to liver cells.

High levels of HDL and apolipoprotien A-1 (ApoA1), the major HDL protein, have long been associated with decreased risk for cardiovascular disease. ApoA1 is a single polypeptide chain with 243 amino acid residues of known primary amino acid sequence (Brewer et al., (1978) Biochem. Biophys. Res. Commun. 80:623-630). ApoA1 acts as an acceptor of cellular cholesterol in the reverse cholesterol transport by mediating cholesterol efflux from cells. Each HDL particle contains at least one copy (and usually two to four copies) of ApoA1. ApoA1 is synthesized in humans in the form of a preproapolipoprotein of 267 residues by the liver and small intestine which is secreted as a proprotein that is rapidly cleaved by the action of a calcium-dependent protease to generate a mature 243 amino acid polypeptide and secreted into the plasma. ApoA1 has been postulated to possess eight tandem repeating 22 mer sequences and two 11 mer sequences, most of which have the potential to form class A amphipathic helical structures (Segrest et al. (1974) FEBS Lett. 38:247-253). Characteristics of the class A amphipathic helix including the presence of positively charged residues at the polar-nonpolar interface and negatively charged residues at the center of the polar face (Segrest et al. (1974) FEBS Lett. 38:247-253; Segrest et al. (1990) Proteins: Structure, Function, and Genetics 8:103-117).

ApoA1 forms three types of stable complexes with lipids: small, lipid-poor complexes referred to as pre-beta-1 HDL; flattened discoidal particles containing polar lipids (phospholipid and cholesterol) referred to as pre-beta-2 HDL; and spherical particles containing both polar and nonpolar lipids, referred to as spherical or mature HDL (HDL₃ and HDL₂). Most HDL in the circulating population contain both ApoA1 and ApoAII (the second major HDL protein) and are referred to as the Al/AII-HDL fraction of HDL. However, the fraction of HDL containing only ApoA1 (referred to herein as A1-HDL fraction) appear to be more effective in RCT. Certain epidemiologic studies support the hypothesis that the A1-HDL fraction is anti-atherogenic. (Parra et al., 1992, Arterioscler. Thromb. 12:701-707; Decossin et al., 1997, Eur. J. Clin. Invest. 27:299-307).

### SUMMARY OF THE INVENTION

The invention is set out in the appended claims. The embodiments of the description which do not fall within the scope of said claims are provided for illustrative purposes only and do not form part of the present invention. The present disclosure provides methods for treating cancer in a subject, as well as related compositions and kits, that employ a therapeutic agent, or a nucleic acid sequence encoding a therapeutic agent, selected from apolipoprotein A-1 (ApoA1) (or biologically active fragment thereof), an ApoA1 mimetic, an agent that increases expression of ApoA1, or a binding agent specific for oxidized ApoA1. In certain examples, the ApoA1 or ApoA1 mimetic is at least partially oxidation resistant.

The present disclosure relates to a method of treating cancer in a subject. In certain examples, the methods include administering to the subject an amount of high density lipoprotein (HDL), apolipoprotien A-1 (ApoA1) (e.g., human ApoA1) or biologically active fragment thereof, and/or ApoA1 mimetic (e.g., ApoA1-Milano, APL180, or D4F peptide) effective to prevent, suppress and/or inhibit cancer cell growth, survival, proliferation, and/or replication in the subject. The HDL, ApoA1, and/or ApoA1 mimetic can be administered in a pharmaceutical composition directly to the subject or administered by delivering an agent or therapeutic that increases the level of HDL, ApoA1, and/or ApoA1 mimetic in the subject.

In some examples, the present disclosure provides methods of treating cancer in a subject comprising: administering to a subject a composition comprising a therapeutic agent and/or a nucleic acid sequence encoding the therapeutic agent, wherein the therapeutic agent comprises: i) apolipoprotein A-1 (ApoA1) or biologically active fragment thereof, and/or ii) an ApoA1 mimetic (e.g., ApoA1-Milano, APL180, or D4F peptide), wherein the administering is effective to prevent, suppress, and/or inhibit cancer cell growth in the subject.

In certain examples, the therapeutic agent comprises a high-density lipoprotein (HDL). In other examples, the therapeutic agent comprises preproapoliprotein (preproApoA1). In other examples, the composition is injected and/or infused into the subject. In additional examples, the nucleic acid sequence comprises an ApoA1 mRNA sequence. In further examples, the nucleic acid sequence comprises an ApoA1 mimetic mRNA sequence. In additional examples, the nucleic acid sequence comprises a DNA sequence encoding the therapeutic agent. In other examples, the nucleic acid sequence comprises an expression vector.

In particular examples, the present disclosure provides methods of treating cancer in a subject comprising: administering to a subject a composition comprising a therapeutic agent, wherein the therapeutic agent induces increased endogenous expression of apolipoprotein A-1 (ApoA1) is the subject effective to prevent, suppress, and/or inhibit cancer cell growth in the subject. In certain examples, the therapeutic agent comprises a small molecule compound. In other examples, the therapeutic agent comprises a nucleic acid sequence encoding the HNF-4 gene or active portion thereof. In further examples, the therapeutic agent comprises a statin that is able to cause an increased expression of HDL or ABCA1. In other examples, the statin comprises Atorvastatin. In other examples, endurance exercise training is prescribed to raise HDL and therefore increase expression of ApoA1. In other examples, the therapeutic agent is niacin. In further examples, the therapeutic agent is a fibrate that is able to cause increased expression of HDL. In other examples, the fibrate is selected from the group consisting of: Fenofibrate, bezafibrate, gemfibrozil, and LY518674.

In certain examples, the present disclosure provides pharmaceutical compositions for treating cancer in a subject comprising: a therapeutically effective amount of a therapeutic agent and/or a nucleic acid sequence encoding the therapeutic agent, wherein the therapeutic agent comprises: i) apolipoprotein A-1 (ApoA1), and/or ii) an ApoA1 mimetic, wherein the therapeutically effective amount is effective to prevent, suppress and/or inhibit cancer cell growth, survival, proliferation, and/or replication in the subject. In other examples, the therapeutic agent comprises a high-density lipoprotein. In additional examples, the therapeutic agent comprises preproapoliprotein (preproApoA1). In some examples, the nucleic acid sequence comprises an ApoA1 mRNA sequence. In particular examples, the nucleic acid sequence comprises an ApoA1 mimetic mRNA sequence. In further examples, the nucleic acid sequence comprises a DNA sequence encoding the therapeutic agent. In other examples, the nucleic acid sequence comprises an expression vector.

In some examples, the present disclosure provides methods of treating cancer in a subject comprising: administering to a subject at least one agent that stimulates the cellular receptors for HDL, the SR-B1 receptor, and/or the cellular cholesterol transporters ABCA1 and ABCG1. In particular examples, the agent comprises at least one of: a small molecule, a peptide mimetic, an antibodies or fragments thereof.

In further examples, the present disclosure provides methods for characterizing a subject's risk for cancer, comprising: a) determining the level of dysfunctional DHL in a subject; and b) comparing the measured level to a control value wherein an increase in the measured level of dysfunctional HDL compared to the control value correlates in the subject with at least one of lack of protection against cancers, development of tumors, tumor metastasis, and shortened survival time of the subject.

In some examples, the present disclosure provides methods of treating or preventing cancer is a subject comprising: administering to a subject a composition comprising a binding agent specific for oxidized ApoA1, wherein the administering is effective to prevent, suppress, and/or inhibit cancer cell growth in the subject. In particular examples, the binding agent comprises an antibody or antigen-binging portion thereof. In other examples, the antibody is the 10C5.2 monoclonal antibody, or an antigen-binding portion of the 10C5.2 monoclonal antibody. In other examples, the binding agent comprises at least one variable region, or at least one CDR, from the 10C5.2 monoclonal antibody. In some examples, the antibody is the 10G1.5 monoclonal antibody, or an antigen-binding portion of the 10G1.5 monoclonal antibody. In further examples, the binding agent comprises at least one variable region, or at least one CDR, from the 10G1.5 monoclonal antibody. In additional examples, the antibody is the 4G11.2 monoclonal antibody, or an antigen-binding portion of the 4G11.2 monoclonal antibody. In other examples, the binding agent comprises at least one variable region, or at least one CDR, from the 4G11.2 monoclonal antibody.

In certain examples, the present disclosure provides methods of treating or preventing cancer is a subject comprising: administering to a subject a composition comprising an HDL mimetic peptide, wherein the administering is effective to prevent, suppress, and/or inhibit cancer cell growth in the subject. In some examples, the HDL mimetic peptide is LSI-518P from Lipid Sciences, Inc. In further examples, the HDL mimetic peptide is mimetic peptide 4F (see Vakili et al., Adv Exp Med Biol. 2010;660:167-72). In certain examples, the HDL mimetic peptide is ATI-5261 (see, Bielicki et al., J Lipid Res. 2010 Jun;51 (6):1496-503). In other examples, the HDL mimetic is as shown in D'Souza et al., Circulation Research. 2010;107:217.

In an aspect of the present disclosure, HDL, ApoA1 and /or ApoA1 mimetic is administered to a subject with or at risk of cancers including but not limited to malignant melanoma, lung, breast, colon, brain, kidney, bladder, prostate, pancreatic, oral, head and neck, sarcomas, lymphomas (B-cell and T-cell) and adrenal cancer. HDL, ApoA1, and/or ApoA1 mimetics can slow development of tumors and tumor metastasis as well as lengthen survival time of the subject.
The present disclosure further relates to a pharmaceutical composition for treating cancer in a subject. The pharmaceutical composition includes a therapeutically effective amount of high density lipoprotein (HDL), apolipoprotien A-1 (ApoA1), and/or ApoA1 mimetic effective to prevent, suppress and/or inhibit cancer cell growth, survival, proliferation, and/or replication in the subject.

Another aspect of the present disclosure relates to a method of treating cancer in a subject by administering to the subject at least on agent that stimulates the cellular receptors for HDL, the SR-B1 receptor, and/or the cellular cholesterol transporters ABCA1 and ABCG1. The agent can comprise at least one of small molecules, peptide mimetics, antibodies or fragments thereof and be administered to the subject to protect the subject against a wide variety of cancers including but not limited to malignant melanoma, lung, breast, colon, brain, kidney, bladder, prostate, pancreatic, oral, head and neck, sarcomas, lymphomas (B-cell and T-cell) and adrenal cancer development as well as slow development of tumors, slow tumor metastasis, and lengthen survival time of the subject.

A further aspect of the present disclosure relates to a diagnostic method for characterizing a subject's risk for cancer. The diagnostic method can include determining the level of dysfunctional HDL in a subject and comparing the measured level to a control value. An increase in the measured level of dysfunctional HDL compared to the control value can correlate with lack of protection against a wide variety of cancers including but not limited to malignant melanoma, lung, breast, colon, brain, kidney, bladder, prostate, pancreatic, oral, head and neck, sarcomas, lymphomas (B-cell and T-cell) and adrenal cancer development; facilitate development of tumors, tumor metastasis and shorten survival times.

### BRIEF DESCRIPTION OF THE DRAWINGS

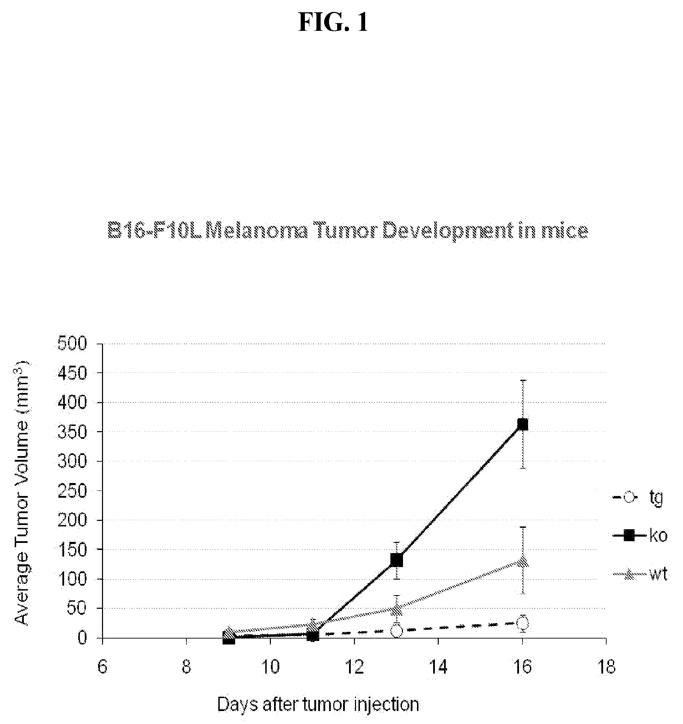

Fig. 1 is a plot showing volume of tumor over time for a B16-F10L melanoma tumor line injected in mice with either low (ApoA1 knockout) or high (human ApoA1 transgenic) HDL levels. Mice (13 animals per genotype; male and female at 9 wks old) were injected subcutaneously on each flank with 1x10⁵ B16-F10L tumor cells. Tumor volume at the site of injection was monitored by caliper measurements. Average tumor volume was derived by adding up the total (both flanks) tumor burden per mouse for each of 13 mice per group and dividing the number by n=26 (total injection sites). Abbreviations are as follows: KO= ApoA1 null on C57B1/6 background, tg= human ApoA1 expressing transgenic C57B1/6, Wt= wild type C57B 1/6.
Fig. 2 is a plot showing survival of mice over time for mice with either low (apoA1 knockout) or high (human apoA1 transgenic) HDL levels injected with B16-F10L melanoma tumor line. Mice (13 animals per genotype; male and female at 9 wks old) were injected subcutaneously on each flank with 1x10⁵ B16-F10L tumor cells. Abbreviations are as follows: KO= ApoA1 null on C57B1/6 background, tg= human ApoA1 expressing transgenic C57B1/6, Wt= wild type C57B 1/6.

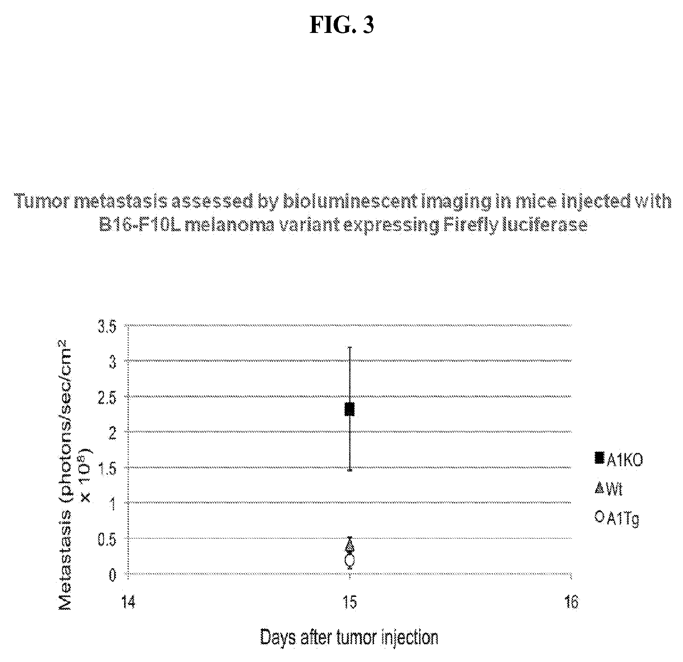

Fig. 3 is a graph showing tumor metastasis assessed by bioluminescent imaging in mice injected with B16-F10L melanoma variant expressing firefly luciferase. Mice (male and female at 9 wks old) were injected subcutaneously on each flank with 1x10⁵B16-F10L_FLuc (firefly luciferase. Melanoma tumor cells and imaged using IVIS (Xenogen) imaging system. Several days prior to imaging mice were anesthetized with a cocktail of ketamine:xylazine (4:1) in PBS and shaved over their entire body to minimize the amount of light absorbed by black fur. To image, mice were injected intraperitoneally with 100 µl 30mg per ml of the luciferase substrate, D-luciferin (Xenogen)) in PBS, anaesthetized in an isoflurane chamber and then imaged for 2 seconds. Ventral images were taken to denote metastasis and quantified using Living Image Software (Xenogen). Error bars denote standard error where n=8 (ApoAIKO), 11 A1Tg and 12 Wt. N.B metastasis in Wt strain is approximately 2-fold greater than in A1Tg.
Fig. 4 is a schematic illustration of biological processes for genes UP-regulated by HDL. Bio-informatic analyses were performed using Ingenuity Pathway Analysis software. There were 99 genes that were up-regulated by HDL. The number of genes associated with each biological process is indicated. Many genes mapped to several processes hence the total number in the pie chart adds up to more than 99 genes.
Fig. 5 is a schematic illustration of biological processes for genes DOWN-regulated by HDL. Bio-informatic analyses were performed using Ingenuity Pathway Analysis software. There were 269 genes that were down-regulated by HDL. The number of genes associated with each biological process is indicated. Many genes mapped to several processes and several other general processes are not included in this pie chart hence the total number in the pie chart does not add up to 269 genes.
Figure 6 shows that gene therapy with apoA1 is protective for cancer development (tumor growth and metastases formation). ApoA1 transgenic mice show reduced tumor burden (Figure 6A), growth rate (Figure 6A), and metastases (Figure 6B) formation relative to wild type (WT) mice. Figure 6 also shows that genetic deletion of apoA1 (apoA1-KO mice) are more susceptible to development of tumor (Figure 6A), growth rate of tumor (Figure 6A), and metastases formation (Figure 6B).
Figure 7A shows the mean tumor volume in mice injected with Lewis lung cancer cells after 48 days, and Figure 7B shows the survival percentage over time.
Figure 8 shows results from Tg, WT, and A1KO mice after being injected with F10L_B16 melanoma tumor cells and monitored for survival.
Figure 9 shows plasma levels of ApoA1 after apoA1 injection (subcutaneously) into mice every other day. Figure 9 shows that the plasma level of apoA1 is between 75-175 mg/dL.
Figure 10 shows results from Example 3 where human ApoA1 or normal saline (control) was given to apoA1-KO who were injected with F10L_B16 Melanoma Tumor cells. Total tumor burden and metastases were quantified by xenogen imaging. Total tumor burden results are shown in Figure 10A, and metastases results are shown in Figure 10B.
Figure 11 shows the same mice described in Figure 10 at 16 days post tumor injection.
Figure 12 shows the same mice described in Figure 10 at 21 days post tumor injection.
Figure 13 shows a comparison by gender of the mice described in Figure 10.
Figure 14 shows survival time of the mice described in Figure 10.
Figure 15 shows the impact of giving apoA1 as a therapy after the tumor growth is established and metastases are detectable. ApoA1 injections were started one week after tumor injection. Overall tumor growth is shown in Figure 16a, and metastases formation is shown in Figure 16B.
Figure 16 shows that ApoA1 is markedly more potent than ApoA2 at reducing cancer growth (Fig. 16A) and metastases formation (Fig. 16B).
Figure 17 shows a comparison of spleen macrophages from B16_F10L tumor-bearing A1KO mice and A1Tg mice. Figure 17 show results including cell number (Fig. 17A) and F480+ cells as a percentage of splenocytes (Fig. 17B).
Figure 18A shows a cell plot of Cd11b-PE vs. GR1-APC for AIKo and A1 Tg derived cells. Figure 18B shows the average percentage of cd1b+/GR1+ (MDSC) cells for A1KO and A1Tg derived cells. Figure 18C shows the average as a percentage of MDSC cells for A1KO and A1Tg cells.
Figure 19 shows the effect of apoA1 on angiogenisis.
Figure 20 shows that animals that lack SRB1 (both heterozygotes and homozygotes) showed protection from induced cancer growth, while the SRB1 containing mice (SRB1+/+) have a greater rate of induced tumor growth.
Figure 21 shows the results of screening various monoclonal antibodies that target different forms of oxapoA1 against malignant melanoma human cancer biopsy specimens.
Figure 22 shows immunohistochemistry on the human melanoma skin and normal skin tissue. The anti-human ox-apoA1 mouse monoclonal antibody 10C5.2 was used at 10 ug/ml. Results are shown in Figure 22, where A-C show ox-apoA1 staining; D-F show mouse IgG1 control staining, and G-I show H&E staining.
Figures 23-26 shows the results of screening one control mAb and three mAbs shown to be specific for oxidized apoA1 against human malignant melanoma tumors. The oxidized apoA1 specific antibodies were each shown to intensely stains multiple human malignant melanoma tumors but not normal biopsy. Figure 23 shows the results for Mab 10G1.5, Figure 24 shows the results for Mab 10C5.2, Figure 25 shows the results for Mab 4611.2, while Figure 26 shows the results of a Mab control antibody.

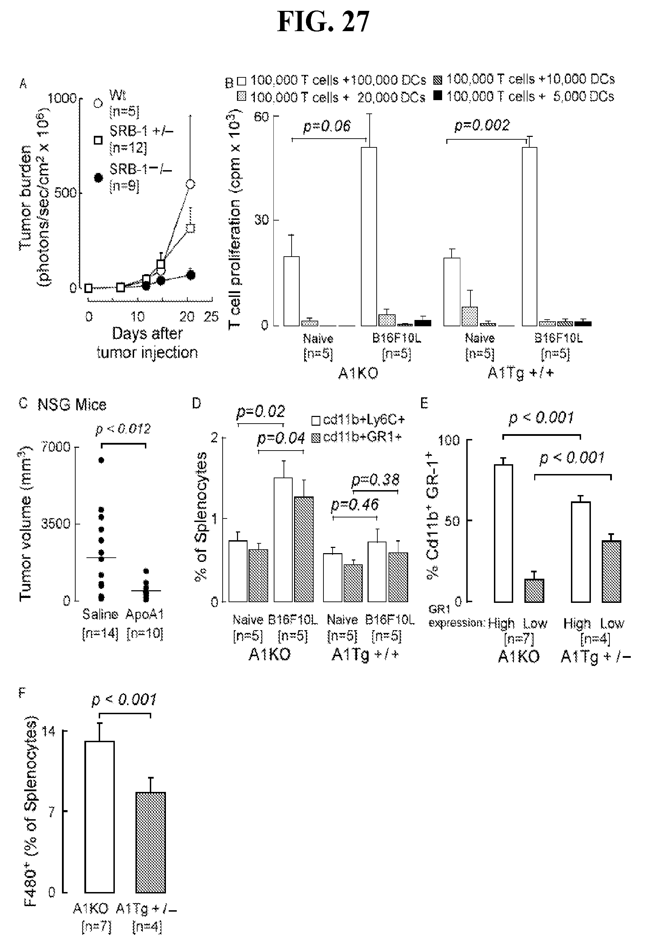

Figure 27A shows the results of Example 4 where mice deficient (SRB-1-/-) or heterozygous (SRB-1+/-) for HDL receptor, SRB-1, were injected with B16F10L-luciferase (1x105 cells s.c., per flank) and tumor growth was monitored by IVIS Xenogen live imaging. Figure 27B shows stimulation of allogenic T cell proliferation by splenic dendritic cells (DCs) isolated from naive or B16F10L-luciferase tumor bearing mice of indicated genotypes (n= number of mice) 14 days post tumor cell injection. Data in Figure 27C, where n= number of tumor injection sites, shows the terminal (day 21 post tumor injection; s.c. bilaterally) tumor volume measurements in B16F10L-luciferase bearing NSG mice injected (s.c., away from the site of tumor injection) every other day starting on day 1 post tumor injection with ApoA1 (20 mg/animal) or normal saline. Flow cytometry was performed on splenocytes from mice (n=number of animals) injected with normal saline (naive), B16F10L-luciferase (1x105 cells s.c., bilaterally) (27D) or injected with B16F10L-luciferase cells at 4 separate sites (1x105 cells s.c per site) on the dorsal side (27E & F)) harvested on day 14 (D) or 12 (E & F) post tumor injection. Data points are mean +/-SEM.

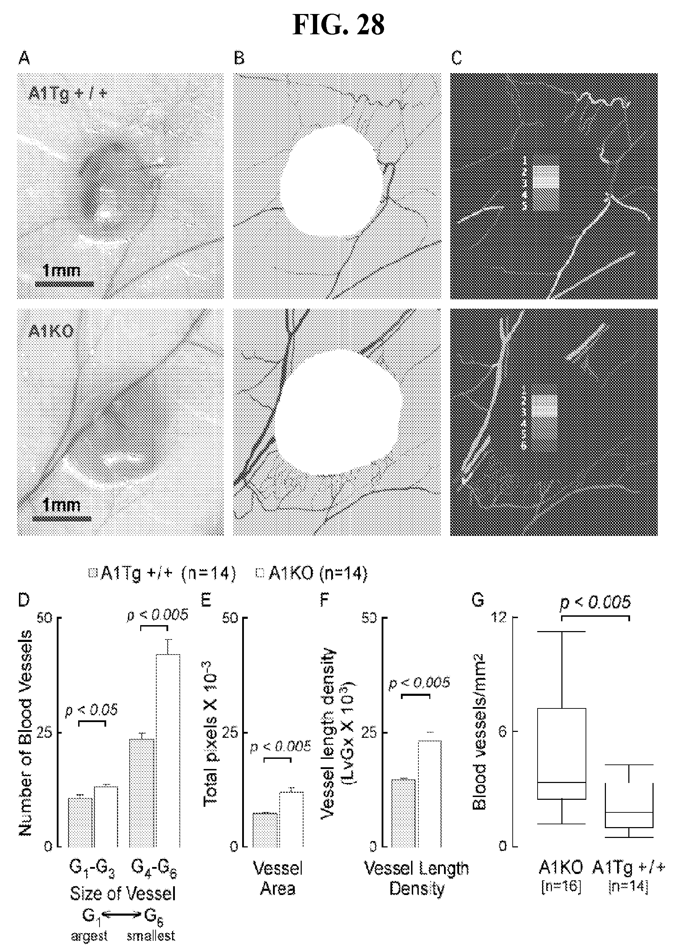

Figure 28 shows the results of Example 4 where angiogenesis was assessed in B16F10L melanoma bearing mice 7 days post tumor injection (s.c.,1x105 cells per flank). Tumor photographs were subjected to VESGEN analysis whereby color photomicrographs (Figure 28A) were converted to input vascular binary images (Figure 28B). The region of interest (ROI) representing the tumor mass (white in B) defined the perimeter of the tumor. The output of VESGEN was a series of color Generation Maps (colored vessels on black background) in which the largest diameter vessels were defined as G1 (red), with each subsequent smaller generation represented as G2 - G6 (C). Data in Figures 28D, E, and F were generated from panel C and show number of blood vessels based on vessel diameter (D), total vessel area (E) and vessel length density (F). (G) Number of vessels feeding directly into the primary tumor was counted under a microscope where n= tumor injection sites.

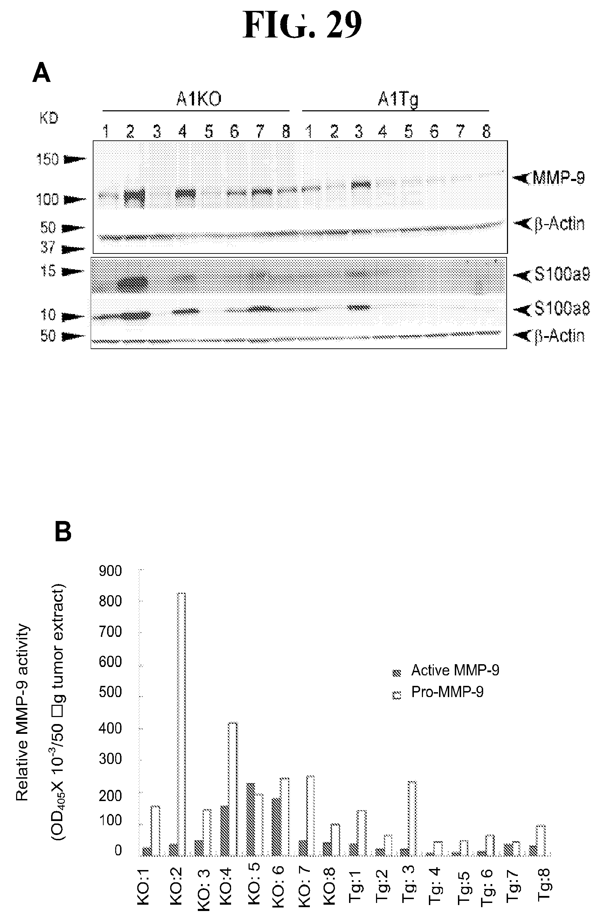

Figure 29 shows results from Example 4 where primary subcutaneous B16F10L tumors were harvested from A1KO and A1Tg tumor bearing mice (8 animals per genotype) 7 days post injection (s.c.,1x105 cells per flank). Total tumor protein extracts were prepared and subjected to Western blotting. Specific protein bands were detected with Odyssey infrared imaging system with actin serving as loading control. Results are shown in Figure 29A. Figure 29B shows MMP-9 activity in tumor extracts (in A) was assayed using Quickzyme assay kit according to the manufacturer's protocol. Pro-MMP-9 refers to total activity observed following chemical activation (APMA). Total activity includes the active enzyme (active MMP-9).
Figure 30 shows that HDL directly inhibits cancer cell migration and tissue invasion potential. Figure 30A shows the results from Example 4 where melanoma cells were exposed to apoA1 and HDL and migration of cells was determined. Figure 30B shows the results of Example 4 where exposed to apoA1 and HLL, and invasion of cells was determined.

### DEFINITIONS

For convenience, certain terms employed in the specification, examples, and appended claims are collected here. Unless defined otherwise, all technical and scientific terms used herein have the same meaning as commonly understood by one of ordinary skill in the art to which this invention belongs.

As used herein, "protein" is a polymer consisting essentially of any of the 20 amino acids. Although "polypeptide" is often used in reference to relatively large polypeptides, and "peptide" is often used in reference to small polypeptides, usage of these terms in the art overlaps and is varied.

The terms "polynucleotide sequence," "nucleic acid sequence," and "nucleotide sequence" are also used interchangeably herein.

"Recombinant," as used herein, means that a protein is derived from a prokaryotic or eukaryotic expression system.

The term "wild type" refers to the naturally-occurring polynucleotide sequence encoding a protein, or a portion thereof, or protein sequence, or portion thereof, respectively, as it normally exists in vivo.

The term "mutant" refers to any change in the genetic material of an organism, in particular a change (*i.e.* deletion, substitution, addition, or alteration) in a wild type polynucleotide sequence or any change in a wild type protein. The term "variant" is used interchangeably with "mutant." Although it is often assumed that a change in the genetic material results in a change of thee function of the protein, the terms "mutant" and "variant" refer to a change in the sequence of a wild type protein regardless of whether that change alters the function of the protein (*e*.*g*., increases, decreases, imparts a new function), or whether that change has no effect on the function of the protein (*e.g*., the mutation or variation is silent). As used herein, the term "nucleic acid" refers to poly nucleotides, such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides.

As used herein, the term "gene" or "recombinant gene" refers to a nucleic acid comprising an open reading frame encoding a polypeptide, including both exon and (optionally) intron sequences.

As used herein, the term "vector" refers to a nucleic acid molecule capable of transporting another nucleic acid to which it has been linked. Preferred vectors are those capable of autonomous replication and/or expression of nucleic acids to which they are linked. Vectors capable of directing the expression of genes to which they are operatively linked are referred to herein as "expression vectors."

A polynucleotide sequence (DNA, RNA) is "operatively linked" to an expression control sequence when the expression control sequence controls and regulates the transcription and translation of that polynucleotide sequence. The term "operatively linked" includes having an appropriate start signal (*e*.*g*., ATG) in front of the polynucleotide sequence to be expressed, and maintaining the correct reading frame to permit expression of the poly nucleotide sequence under the control of the expression control sequence, and production of the desired polypeptide encoded by the polynucleotide sequence.

As used herein, the term "tissue-specific promoter" means a nucleic acid sequence that serves as a promoter, and which affects expression of the selected nucleic acid sequence operably linked to the promoter, and which affects expression of the selected nucleic acid sequence in specific cells of a tissue, such as cells of epithelial cells. The term also covers so-called "leaky" promoters, which regulate expression of a selected nucleic acid primarily in one tissue, but cause expression in other tissues as well.

"Homology" and "identity" are used synonymously throughout and refer to sequence similarity between two peptides or between tow nucleic acid molecules. Homology can be determined by comparing a position in each sequence, which may be aligned for purposes of comparison. When a position in the compared sequence is occupied by the same base or amino acid, then the molecules are homologous or identical at that position. A degree of homology or identity between sequences is a function of the number of matching or homologous positions shared by the sequences.

A "chimeric protein" or "fusion protein" is a fusion of a first amino acid sequence encoding a polypeptide with a second amino acid sequence defining a domain (*e*.*g*., polypeptide portion) foreign to and not substantially homologous with any domain of the first polypeptide. A chimeric protein may present a foreign domain which is found (albeit in a different protein) in an organism which also expresses the first protein, or it may be an "interspecies," "intergenic," etc. fusion of protein structures expressed by different kind of organisms.

The phrases "parenteral administration" and "administered paternterally" as used herein means modes of administration other than enteral and topical administration, usually by injection, and includes, without limitation, intravenous, intramuscular, intraaterial, intrathecal, intraventricular, intracapsular, intraorbital, intracarida, intradermal, intraperitoneal, transtracheal, subcutaneous, subcuticular, intraarticular, subcapsular, subarachnoid, intraspinal and transternal injection and infusion. The compositions of the present invention may be administered by parenteral administration.

The phrases "systemic administration," administered systemically," "peripheral administration," and "administered peripherally" as used herein mean the administration of a compound, drug or other material other than directly into the central nervous system, such that it enters the animal's system and, thus, is subject to metabolism and other like processes, for example, subcutaneous administration. The compositions of the present invention may be administered by systemic administration.

### DETAILED DESCRIPTION

The invention is set out in the appended claims. The embodiments of the description which do not fall within the scope of said claims are provided for illustrative purposes only and do not form part of the present invention. The present disclosure relates to compositions and methods of treating and/or preventing cancer in a subject. During the development of certain examples of the present disclosure, it was found that administration of HDL, ApoA1, and/or ApoA1 mimetics to a subject can prevent, suppress and/or inhibit cancer survival and cancer cell proliferation as well as inhibit and/or suppress multiple pathways linked to cancer growth and replication. An aspect of the present disclosure therefore relates to a method of treating or preventing cancer in a subject by administering to the subject an amount of high density lipoprotein (HDL), apolipoprotein A-1 (ApoA1) or a biologically active fragment thereof, and/or ApoA1 mimetic effective to prevent, suppress and/or inhibit cancer cell growth, survival, proliferation, and/or replication in the subject.

High density lipoprotein (HDL) administered to the subject in accordance with the present disclosure can include a lipid-protein complex or derivative thereof which when isolated from plasma by ultracentrifugation is found in the density range of d=1.063 to d=1.21. In one example of the present disclosure, HDL can be isolated from human plasma and administered to a subject to protect against a wide variety of cancers, such as malignant melanoma, lung, breast, colon, brain, kidney, bladder, prostate, pancreatic, oral, head and neck, sarcomas, lymphomas (B-cell and T-cell) and adrenal cancer development; as well as to slow development of tumors, tumor metastasis and lengthens survival times. Preferably, the HDL or derivative thereof is a peptide or protein derivative of the sequence of ApoA1, or a peptide or protein mimetic functionally homologous to the active portions of ApoA1.

In one example, the HDL used in accordance with the present disclosure is reconstituted HDL. The term "reconstituted HDL" means HDL composed of a lipid or lipids in association with at least one of the apolipoproteins of HDL. The components may be derived, for example, from blood, or produced by recombinant technology. For example, nascent HDL can be prepared from ApoA1 isolated from human plasma combined with a wide-range of phospholipids, such as from 5-100:1 molar ration of phospholipids to apoAI, and reconstituted into nascent HDL particle that can be administered to a subject to treat or prevent cancer. In another example, HDL can include monomeric and multimeric HDL peptide mimetics that can function to allow HDL remodeling leading to greater anti-inflammatory activity and cholesterol efflux activity.

ApoA1 administered in accordance with the present disclosure can include a full-length human ApoA1 peptide or to a fragment or domain thereof comprising a class A amphipathic helix. In some examples, ApoA1 can be combined with a wide-range of phospholipids, such as from 5-100:1 molar ratio of phospholipids to ApoA1, reconstituted into a nascent HDL particle, and administered to a subject to treat or prevent cancer. The ApoA1 reconstituted into a HDL particle and administered to a subject can be protective against a wide variety of cancers including, malignant melanoma, lung, breast, colon, brain, kidney, bladder, prostate, pancreatic, oral, head and neck, sarcomas, lymphomas (B-cell and T-cell) and adrenal cancer development, slow development of tumors, and lengthens survival times of the subject.

In other examples, ApoA1 can be administered in combination with a wide-range of phospholipids and cholesterol, such as from 5-100:1 molar ratio of phospholipids:cholesterol:ApoA1 and reconstituted into a nascent HDL cholesterol particle. The ApoA1 reconstituted into a HDL cholesterol particle and administered to a subject is protective against a wide variety of cancers including, malignant melanoma, lung, breast, colon, brain, kidney, bladder, prostate, pancreatic, oral, head and neck, sarcomas, lymphomas (B-cell and T-cell) and adrenal cancer development, slow development of tumors, and lengthens survival times of the subject.

The ApoA1 administered to a subject to treat cancer can also include ApoA1 mimetics. The terms "mimetics of ApoA1" or "known mimetics of ApoA1," or "ApoA1 mimetics," refer to mimetics of ApoA1 that can be identified or derived from any reference and that have ApoA1 behavior. These include mimetics of ApoA1 identified in U.S. and foreign patents and publications.

ApoA1 mimetics can include natural variants of ApoA1 that are known in the art. For example, Weisgraber et al. has shown that cysteine can be substituted for arginine at position 173 in a mutant ApoA1 termed ApoA1-Milano (Weisgraber et al. (1983) J. Biol. Chem. 258:2508-2513). ApoA1 polypeptide mimetics contemplated in the present disclosure can also include polypeptides from the ApoA1 forms and variants including, for example, apolipoprotein A-1 (Brewer et al., (1978)), apolipoprotein A-1 Milano (Weisgraber (1983)), apolipoprotein A-1 Paris (Bielicki and Oda (2002) Biochemistry 41:2089-2096), proapolipoprotein A-1, or any other mutant form of ApoA1 known in the art whether synthetically formed or naturally occurring.

Alternatively, the ApoA1 mimetics of the present disclosure can include an amphipathic helical peptides that closely mimic the class A amphipathic helix of human or mouse ApoA1 peptide (*i.e.,* mimetics of ApoA1). The term "an amphipathic helical peptide" refers to a pepti de comprising at least on amphipathic helix (amphipathic helical domain). Certain amphipathic helical peptides of this invention can comprise two or more *(e.g.,* 3, 4, 5, etc.) amphipathic helices.

The term "class A amphipathic helix" refers to a protein structure that forms in an a-helix producing a segregation of a polar and nonpolar faces with the positively charged residues residing at the polar-nonpolar interface and the negatively charged residues residing at the center of the polar face (see, *e.g.,* Segrest et al. (1990) Proteins: Structure, Function, and Genetics 8:103-117). Particularly preferred peptides may include greater than about 50% amino acid sequence identity with the polypeptide encoded by the exon encoding a class A amphipathic helix of human or mouse ApoA1. The peptide may be combined with a pharmacologically acceptable excipient (*e*.*g*., an excipient suitable for oral administration to a mammal).
In one embodiment, the ApoA1 mimetic can be resistant to oxidation when administered to a subject. By "resistant to oxidation" or "oxidant resistant" as used in the specification and the claims, it is meant the ApoA1 mimetic according to the present invention has an amino acid sequence that is substantially similar to the amino acid sequence of ApoA1, ApoA1 fragments, or known mimetics of ApoA1 that contain at least on tryptophan and were at least on tryptophan residues is substituted with oxidant resistant residues, such as an oxidant resistant peptide residue, and for which ApoA1 lipid binding an efflux activities are retained.

In another example, the oxidant resistant residue of the ApoA1 mimetic can include an aromatic peptide residue, such as phenylalanine. Examples of oxidant resistant ApoA1 mimetics are disclosed in U.S. Patent Application No. 12/256,822, filed October 23, 2008. For example, the oxidant resistant ApoA1 mimetic can include forms of human ApoA1, such as 4WF (tryptophans [W] at amino acids 8, 50, 72 and 108 mutated to phenylalanines [F]).

In another example, the oxidant resistant ApoA1 mimetic used to treat cancer in a subject can fail to activate a proinflammatory cellular reaction including activation of nuclear factor kappa B transcription factor, or vascular cell adhesion molecule (VCAM) expression. For example, it was found that oxidant resistant forms of human ApoA1 that include 166 tyrosine (y) to glutamic acid (E) or aspartic acid (D) mutations retain Lecithin-cholesterol acyltransferase (LCAT) binding and are capable of stimulation LCAT activity as well as fail to activate a proinflammatory cellular reaction including activation of nuclear factor kappa B transcription factor, or vascular cell adhesion molecule (VCAM) expression.

In some examples of the present disclosure, the oxidant resistant ApoA1 can include the 4WF mutations in combination with Y166 E or D mutations and be administered to a subject to protect against a wide variety of cancers including, malignant melanoma, lung, breast, colon, brain, kidney, bladder, prostate, pancreatic, oral, head and neck, sarcomas, lymphomas (B-cell and T-cell) and adrenal cancer development as well as slow development of tumors, slow tumor metastasis, and lengthen survival times of the subject.

In certain examples of the present disclosure, the ApoA1 mimetic have the following amino acid sequence: wherein X is either a tryptophan residue or an oxidant resistant residue (e.g., phenylalanine) and at least one of the four X's is an oxidant resistant residue. In other examples, at least two of the Xs of SEQ ID NO: 1 are an oxidant resistant residue, at least three of the Xs of SEQ ID NO: 1 are oxidant resistant residues, or all four of the Xs are oxidant resistant residues.

In certain examples, the ApoA1 mimetics, besides including tryptophan substituted wild-type or native forms of ApoA 1, can also include tryptophan substituted natural variants of ApoA1 that are known in the art. For example, Weisgraber et al. has shown that cysteine can be substituted for arginine at position 173 in a mutant ApoA1 termed ApoA1-Milano (Weisgraber et al. (1983) J. Biol. Chem. 258: 2508-2513). An ApoA1 mimetic based on ApoA1-Milano can therefore include the amino sequence of SEQ ID NO: 2. wherein X is a tryptophan or an oxidant resistant residue (e.g., phenylalanine) and at least one X is substituted for an oxidant resistant residue.

In another examples of ApoA1 mimetic according to the present in disclosure is based on a known full-length mimetic of human ApoA1 peptide possessing a cysteine residue at position 151 of the mature ApoA1 (corresponding to position 175 in the sequence SEQ ID NO: 3). The ApoA1 mimetic in accordance with this example can include the amino acid sequence of SEQ ID NO: 3. wherein X is a tryptophan or an oxidant resistant residue (e.g., phenylalanine) and at least one X is substituted for an oxidant resistant residue (e.g., phenylalanine).

In some examples, the ApoA1 polypeptide mimetics contemplated in the present disclosure may include modified polypeptides from the ApoA1 forms and variants including, for example, apolipoprotein A-1 (Brewer et al., (1978)), apolipoprotein A-1 Milano (Weisgraber,(1983)), apolipoprotein A-1 Marburg, (Utermann et al., (1982) J. Biol. Chem. 257: 501-507), apolipoprotein A-1 Paris (Bielicki and Oda (2002) Biochemistry 41, 2089-2096), proapolipoprotein A-1, or any other mutant form of ApoA1 known in the art whether synthetically formed or naturally occurring.

In other examples, the ApoA1 mimetics of the present disclosure can include an amphipathic helical peptides that closely mimic the class A amphipathic helix of human or mouse ApoA1 peptide (i.e., mimetics of ApoA1), wherein residues denoted by X can include a tryptophan residue or an oxidant resistant amino acid residue and at least one X is an oxidant resistant residue. The term "an amphipathic helical peptide" refers to a peptide comprising at least one amphipathic helix (amphipathic helical domain). Certain amphipathic helical peptides of this invention can comprise two or more (e.g., 3, 4, 5, etc.) amphipathic helices.

The term "class A amphipathic helix" refers to a protein structure that forms an a-helix producing a segregation of a polar and nonpolar faces with the positively charged residues residing at the polar-nonpolar interface and the negatively charged residues residing at the center of the polar face (see, e.g., Segrest et al. (1990) Proteins: Structure, Function, and Genetics 8: 103-117). Particularl peptides may include greater than about 50% amino acid sequence identity with the polypeptide encoded by the exon encoding a class A amphipathic helix of human or mouse ApoA1. The peptide may be combined with a pharmacologically acceptable excipient (e.g. an excipient suitable for oral administration to a mammal).

In certain examples, the ApoA1 mimetic is a biologically active fragment (e.g., reduces tumor size or other symptoms of cancer) or mimetic of ApoA1, which is capable of treating cancer (e.g., causing tumor size reduction), and comprises or consists of one or more of the following amino acid sequences:
D-X-L-K-A-F-Y-D-K-V-A-E-K-L-K-E-A-F-, (SEQ ID NO: 4)
D-X-F-K-A-F-Y-D-K-V-A-E-K-L-K-E-A-F-, (SEQ ID NO: 5)
D-X-L-K-A-F-Y-D-K-V-A-E-K-F-K-E-A-F-, (SEQ ID NO: 6)
D-X-F-K-A-F-Y-D-K-V-A-E-K-F-K-E-A-F-, (SEQ ID NO: 7)
D-X-L-K-A-F-Y-D-K-V-F-E-K-F-K-E-F-F-, (SEQ ID NO: 8)
D-X-L-K-A-F-Y-D-K-F-F-E-K-F-K-E-F-F-, (SEQ ID NO: 9)
D-X-F-K-A-F-Y-D-K-F-F-E K-F-K-E-F-F-, (SEQ ID NO: 10)
D-X-L-K-A-F-Y-D-K-V-F-E-K-F-K-E-A-F-, (SEQ ID NO: 11)
D-X-L-K-A-F-Y-D-K-V-F-E-K-F-K-E-A-F-, (SEQ ID NO: 12)
D-X-L-K-A-F-Y-D-K-V-F-E-K-L-K-E-F-F-, (SEQ ID NO: 13)
D-X-L-K-A-F-Y-D-K-V-A-E-K-F-K-E-F-F-, (SEQ ID NO: 14)
D-X-L-K-A-F-Y-D-K-V-F-E-K-F-K-E-F-F-, (SEQ ID NO: 15)
E-X-L-K-L-F-Y-E-K-V-L-E-K-F-K-E-A-F-, (SEQ ID NO: 16)
E-X-L-K-A-F-Y-D-K-V-A-E-K-F-K-E-A-F-, (SEQ ID NO: 17)
E-X-L-K-A-F-Y-D-K-V-A-E-K-L-K-E-F-F-, (SEQ ID NO: 18)
E-X-L-K-A-F-Y-D-K-V-F-E-K-F-K-E-A-F-, (SEQ ID NO: 19)
E-X-L-K-A-F-Y-D-K-V-F-E-K-L-K-E-F-F-, (SEQ ID NO: 20)
E-X-L-K-A-F-Y-D-K-V-A-E-K-F-K-E-F-F-, (SEQ ID NO: 21)
E-X-L K-A-F-Y-D-K-V-F-E-K-F-K-E-F-F-, (SEQ ID NO: 22)
D-X-L-K-A-L-Y-D-K-V-A-E-K-L-K-E-A-L-, (SEQ ID NO: 23)
D-X-F-K-A-F-Y-E-K-V-A-E-K-L-K-E-F-F-, (SEQ ID NO: 24)
D-X-F-K-A-F-Y-E-K-F-F-E-K-F-K-E-F-F-, (SEQ ID NO: 25)
E-X-L-K-A-L-Y-E-K-V-A-E-K-L-K-E-A-L-, (SEQ ID NO: 26)
E-X-L-K-A-F-Y-E-K-V-A-E-K-L-K-E-A-F-, (SEQ ID NO: 27)
E-X-F-K-A-F-Y-E-K-V-A-E-K-L-K-E-F-F-, (SEQ ID NO: 28)
E-X-L-K-A-F-Y-E-K-V-F-E-K-F-K-E-F-F-, (SEQ ID NO: 29)
E-X-L-K-A-F-Y-E-K-F-F-E-K-F-K-E-F-F-, (SEQ ID NO: 30)
E-X-F-K-A-F-Y-E-K-F-F-E-K-F-K-E-F-F-, (SEQ ID NO: 31)
D-F-L-K-A-X-Y-D-K-V-A-E-K-L-K-E-A-X-, (SEQ ID NO: 32)
E-F-L-K-A X-Y-E-K-V-A-E-K-L-K-E-A-X-, (SEQ ID NO: 33)
D-F-X-K-A-X-Y-D-K-V-A-E-K-L-K-E-X-X-, (SEQ ID NO: 34)
E-F-X-K-A-X-Y-E-K-V-A-E-K-L-K-E-X-X-, (SEQ ID NO: 35)
D-K-L-K-A-F-Y-D-K-V-F-E-X-A-K-E-A-F-, (SEQ ID NO: 36)
D-K-X-K-A-V-Y-D-K-F-A-E-A-F-K-E-F-L-, (SEQ ID NO: 37)
E-K-L-K-A-F-Y-E-K-V-F-E-X-A-K-E-A-F-, (SEQ ID NO: 38)
E-K-X-K-A-V-Y-E-K-F-A-E-A-F-K-E-F-L-, (SEQ ID NO: 39)
D-X-L-K-A-F-V-D-K-F-A-E-K-F-K-E-A-Y-, (SEQ ID NO: 40)
E-K-X-K-A-V-Y-E-K-F-A-E-A-F-K-E-F-L-, (SEQ ID NO: 41)
D-X-L-K-A-F-V-Y-D-K-V-F-K-L-K-E-F-F-, (SEQ ID NO: 42)
E-X-L-K-A-F-V-Y-E-K-V-F-K-L-K-E-F-F-, (SEQ ID NO: 43)
D-X-L-R-A-F-Y-D-K-V-A-E-K-L-K-E-A-F-, (SEQ ID NO: 44)
E-X-L-R-A-F-Y-E-K-V-A-E-K-L-K-E-A-F-, (SEQ ID NO: 45)
D-X-L-K-A-F-Y-D-R-V-A-E-K-L-K-E-A-F-, (SEQ ID NO: 46)
E-X-L-K-A-F-Y-E-R-V-A-E-K-L-K-E-A-F-, (SEQ ID NO: 47)
D-X-L-K-A-F-Y-D-K-V-A-E-R-L-K-E-A-F-, (SEQ ID NO: 48)
E-X-L-K-A-F-Y-E-K-V-A-E-R-L-K-E-A-F-, (SEQ ID NO: 49)
D-X-L-K-A-F-Y-D-K-V-A-E-K-L-R-E-A-F-, (SEQ ID NO: 50)
E-X-L-K-A-F-Y-E-K-V-A-E-K-L-R-E-A-F-, (SEQ ID NO: 51)
D-X-L-K-A-F-Y-D-R-V-A-E-R-L-K-E-A-F-, (SEQ ID NO: 52)
E-X-L-K-A-F-Y-E-R-V-A-E-R-L-K-E-A-F-, (SEQ ID NO: 53)
D-X-L-R-A-F-Y-D-K-V-A-E-K-L-R-E-A-F-, (SEQ ID NO: 54)
E-X-L-R-A-F-Y-E-K-V-A-E-K-L-R-E-A-F-, (SEQ ID NO: 55)
D-X-L-R-A-F-Y-D-R-V-A-E-K-L-K-E-A-F-, (SEQ ID NO: 56)
X-L-R-A-F-Y-E-R-V-A-E-K-L-K-E-A-F-, (SEQ ID NO: 57)
D-X-L-K-A-F-Y-D-K-V-A-E-R-L-R-E-A-F-, (SEQ ID NO: 58)
E-X-L-K-A-F-Y-E-K-V-A-E-R-L-R-E-A-F-, (SEQ ID NO: 59)
D-X-L-R-A-F-Y-D-K-V-A-E-R-L-K-E-A-F-, (SEQ ID NO: 60)
E-X-L-R-A-F-Y-E-K-V-A-E-R-L-K-E-A-F-, (SEQ ID NO: 61)
D-X-L-K-A-F-Y-D-K-V-A-E-K-L-K-E-A-F-P-D-X-L-K-A-F-Y-D-K-V-A-E-K-L-K-E-A-F, (SEQ ID NO: 62)
D-X-L-K-A-F-Y-D-K-V-A-E-K-L-K-E-F-F-P-D-X-L-K-A-F-Y-D-K-V-A-E-K-L-K-E-F-F, (SEQ ID NO: 63)
D-X-F-K-A-F-Y-D-K-V-A-E-K-L-K-E-A-F-P-D-X-F-K-A-F-Y-D-K-V-A-E-K-L-K-E-A-F, (SEQ ID NO: 64)
D-K-L-K-A-F-Y-D-K-V-F-E-X-A-K-E-A-F-P-D-K-L-K-A-F-Y-D-K-V-F-E-X-L-K-E-A-F, (SEQ ID NO: 65)
D-K-X-K-A-V-Y-D-K-F-A-E-A-F-K-E-F-L-P-D-K-X-K-A-V-Y-D-K-F-A-E-A-F-K-E-F-L, (SEQ ID NO: 66)
D-X-F-K-A-F-Y-D-K-V-A-E-K-F-K-E-A-F-P-D-X-F-K-A-F-Y-D-K-V-A-E-K-F-K-E-A-F, (SEQ ID NO: 67)
D-X-L-K-A-F-V-Y-D-K-V-F-K-L-K-E-F-F-P-D-X-L-K-A-F-V-Y-D-K-V-F-K-L-K-E-F-F, (SEQ ID NO: 68)
and D-X-L-K-A-F-Y-D-K-F-A-E-K-F-K-E-F-F-P-D-X-L-K-A-F-Y-D-K-F-A-E-K-F-K-E-F-F; (SEQ ID NO: 69)
wherein X is a tryptophan or an oxidant resistant residue (e.g., phenylalanine) and at least one X in each sequence is substituted for an oxidant resistant residue.

It will be appreciated that biologically functional equivalents, or even improvements, of the HDL, ApoA1, ApoAlmimetics can be made, generally using ApoA1 as a starting point. Modifications and changes may be made in the structure of such a protein and still obtain a molecule having like or otherwise desirable characteristics. For example, certain amino acids may be substituted for other amino acids in the protein structure without appreciable loss of activity.

It is also well understood by the skilled artisan that, inherent in the definition of a "biologically functional equivalent or fragment" protein or polypeptide, is the concept that there is a limit to the number of changes that may be made within a defined portion of the molecule and still result in a molecule with an acceptable level of equivalent biological activity. Biologically functional equivalent proteins and peptides are thus defined herein as those proteins and peptides in which certain, not most or all, of the amino acids may be substituted. Of course, a plurality of distinct proteins/peptides with different substitutions may easily be made and used in accordance with the present disclosure.

Amino acid substitutions are generally based on the relative similarity of the amino acid side-chain substituents, for example, their hydrophobicity, hydrophilicity, charge, size, and the like. An analysis of the size, shape and type of amino acid side-chain substituents reveals that arginine, lysine, and histidine are all positively charged residues; that alanine, glycine and serine are all a similar size. Therefore, based upon these considerations, arginine, lysine and histidine; alanine, glycine and serine are defined herein as biologically functional equivalents.
Following the procedures noted in the published application by Alton *et al.* (WO83/04053), one can readily design and manufacture genes coding for microbial expression of polypeptides having primary conformations which differ from that herein specified in terms of the identity or location of one or more residues (*e*.*g*. substitutions, terminal and intermediate additions and deletions). Alternately, modifications of cDNA and genomic genes may be readily accomplished by well-known site-directed mutagenesis techniques and employed to generate analogs and derivatives of ApoA1. Such products would share at least one of the biological properties of ApoA1 but may differ in others.

Fragments of ApoA1 or fragments of ApoA1 mimetic that are biologically active with respect to treating cancer can be identified by one of skill in the art. For example a given fragment or mimetic (or mimetic fragment) of ApoA1 can be substituted for the ApoA1 in the Examples below. In this regard, these Examples can be re-run to determine if the fragment or mimetic has the same or better biological activity as ApoA1 as it relates to successfully treating cancer. Other assays, besides those detailed in the Examples, can also be run using the enormous number of cancer compound screening assays known in the art. For example, a candidate fragment of ApoA1 or mimetic can simply be substituted for the test cancer compound in these prior art assays, found, for example, in both journal articles and patents. One could, for example, do a search in PubMed or the Patent databases for "cancer screening assay" and then employ one or more of the assays found in these publications or patents.

The HDL, ApoA1, and/or ApoA1 mimetics of the present disclosure may be purified and isolated. The term "purified and isolated" herein means substantially free of unwanted substances so that the present lipoproteins can be used to treat cancer. For example, one may have a modified recombinant human ApoA1 mimetic polypeptide substantially free of other human proteins or pathological agents. These polypeptides are also characterized by being a product of mammalian cells, or the product of chemical synthetic procedures or of prokaryotic or eukaryotic host expression (*e*.*g*., by bacterial, yeast, higher plant, insect and mammalian cells in culture) of exogenous DNA sequences obtained by genomic or cDNA cloning or by gene synthesis. The products of expression in typical yeast (*e.g., Saccharomyces cerevisiae*) or prokaryote (e.g., *E. coli*) host cells are free of association with any mammalian proteins. The products of expression in vertebrate (*e.g.,* non-human mammalian (*e.g.,* COS or CHO) and avian) cells are free of association with any human proteins. Depending upon the host employed, and other factors, polypeptides of the invention may be glycosylated with mammalian or other eucaryotic carbohydrates or may be non-glycosylated. Polypeptides of the invention may also include an initial methionine amino acid residue (at position-1 with respect to the first amino acid residue of the polypeptide).

The lipoproteins and peptides of the present disclosure can be purified by art-known techniques such as reverse phase chromatography high performance liquid chromatography, ion exchange chromatography, gel electrophoresis, affinity chromatography and the like. The actual conditions used to purify a particular peptide will depend, in part, on the synthesis strategy and on factors, such as net charge, hydrophobicity, hydrophilicity, etc., and will be apparent to those having skill in the art. Multimeric branched peptides can be purified, *e.g.,* by ion exchange or size exclusion chromatography.

For affinity chromatography purification, any antibody which specifically binds the peptide may be used. For the production of antibodies, various host animals, including but not limited to rabbits, mice, rats, etc., may be immunized by injection with a peptide. The peptide may be attached to a suitable carrier, such as BSA, by means of a side chain functional group or linkers attached to a side chain functional group. Various adjuvants may be used to increase the immunological response, depending on the host species, including but not limited to Freund's (complete and incomplete), mineral gels such as aluminum hydroxide, surface active substances such as hemocyanin, dinitrophenol, and potentially useful human adjuvants such as BCG (bacilli Calmette-Guerin) and *Corynebacterium parvum.*

Cancers and related disorders that can be prevented, treated, or managed by methods and compositions of the present disclosure include but are not limited to the following cancers: leukemias, such as but not limited to, acute leukemia, acute myeloid leukemia (AML), chronic myelogenous (or myeloid) leukemia (CML), acute lymphocytic leukemia, acute myelocytic leukemias, such as, myeloblastic, promyelocytic, myclomonocytic, monocytic, and erythroleukemia leukemias and myelodysplastic syndrome; chronic leukemias, such as but not limited to, chronic myelocytic (granulocytic) leukemia, chronic lympocytic leukemia, hairy cell leukemia, polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochrmocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but not limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to squamous cell carcinoma, and adenocarcinoma; uterine cancers such as but not limited to endometrial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcionomas such as but not limited to pappilary, nodular, and diffuse; lung cancers such as non-small cell lung cancer, squamous cell carcinoma (epidermoid carcinoma), adenocarcinoma, large-cell carcinoma, and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenocystic carcinoma; pharynx cancers such as but not limited to squamous cell cancer, and verrucous; skin cancers such as but not limited to basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypemephroma, fibrosarcoma, transitional cell cancer (renal pelvis and/or uterer); Wilms' tumor; bladder cancers such as but not limited to transitional cell carcinoma, squamous cell cancer, adenocarcinoma, carcinosarcoma. In addition, cancers include myxsarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America)

Accordingly, the compositions and methods of the present disclosure are useful in the treatment or prevention of a variety of cancers or other abnormal proliferative diseases, including (but not limited to) the following: carcinoma, including that of the bladder, breast, prostate, rectal, colon, kidney, liver, lung, ovary, pancreas, stomach, cervix, thyroid and skin; including squamous cell carcinoma; hematopoictic tumors of myeloid lineage, including acute and chronic myelogenous leukemias and promoclocytic leukemia; tumors of mesenchymal origin; including fibrosarcoma and rhabdomyoscarcoma; other tumors, including melanoma, seminoma, tetratocarcinoma, neuroblastoma, and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. It is also contemplated that cancers caused by aberrations in apoptosis would also be treated by the methods and compositions of the present disclosure. Such cancers may include but not be limited to follicular lymphomas, hormone dependent tumors of the breast, prostate and ovary, and precancerous lesions such as familial adenomatous polyposis, and myelodysplastic syndromes. In specific examples, malignancy or dysproliferative changes (such as metaplasis and dyplasias), or hyperproliferative disorders, are treated in the skin, lung, colon, rectum, breast, prostate, bladder, kidney, pancreas, ovary, or uterus. In other specific examples, sarcoma, melanoma, small lung carcinoma, or leukemia is treated.

In some examples, the cancer is malignant. In other examples, the disorder to be treated is a pre-cancerous condition. In a specific example, the pre-cancerous condition is high-grade prostatic intraepithelial neoplasia (PIN), fibroadenoma of the breast, or fibrocystic disease.

In certain examples, the compositions of the present disclosure can be delivered to cancer cells by site-specific means. Cell-type specific delivery can be provided by conjugating a therapeutic agent to a targeting molecule, for example, one that selectively binds to the affected cells. Methods for targeting include conjugates, such as those described in U.S. Patent No. 5,391,723. Targeting vehicles, such as liposomes, can be used to deliver a compound, for example, by encapsulating the compound in a liposome containing a cell-specific targeting molecule. Methods for targeted delivery of compounds to particular cell types are well-known to those skilled in the art.

In certain examples, compositions of the present disclosure can be administered together (simultaneously) or at different times (sequentially). In addition, therapy by administration of one or more HDL, ApoA1, and/or ApoA1 mimetics (or other compositions of the present disclosure) can be combined with the administration of one or more therapies such as, but not limited to, chemotherapies, radiation therapies, hormonal therapies, and/or biological therapies/immunotherapies. Prophylactic/therapeutic agents include but are not limited to, proteinaceous molecules, including, but not limited to, peptides, polypeptides, proteins, including post-translationally modified proteins, antibodies, etc.; or small molecules (less than 1000 daltons), inorganic or organic compounds; or nucleic acid molecules, including but not limited to, double-stranded or single-stranded DNA, or double-stranded or single-stranded RNA, as well as triple helix nucleic acid molecules. Prophylactic/therapeutic agents can be derived from any known organism (including, but not limited to, animals, plants, bacteria, fungi, and protista, or viruses) or from a library of synthetic molecules.

The present disclosure also encompasses the use of HDL, ApoA1, and/or ApoA1 mimetics (and other compositions of the present disclosure) for the manufacture of a medicament for the treatment of cancers, such as malignant melanoma, lung, breast, colon, kidney, bladder, prostate, pancreatic, oral, head and neck, sarcomas, lymphomas (B-cell and T-cell) and adrenal cancer or tumors or other cancers or tumor metastases. The present disclosure also encompasses a pharmaceutical composition that includes HDL, ApoA1, and/or ApoA1 mimetics in combination with a pharmaceutically acceptable carrier.

The pharmaceutical composition can include a pharmaceutically acceptable carrier and a non-toxic therapeutically effective amount of HDL, ApoA1, and/or ApoA1 mimetics, or other compositions of the present disclosure.

Moreover, the present disclosure encompasses a pharmaceutical composition for the treatment of disease, the use of which results in the inhibition of growth of neoplastic cells, benign or malignant tumors, or metastases, comprising a pharmaceutically acceptable carrier and a non-toxic therapeutically effective amount of HDL, ApoA1, and/or ApoA1 mimetics, or other compositions of the present disclosure.

The phrases "pharmaceutically or pharmacologically acceptable" refer to molecular entities and compositions that do not produce an adverse, allergic, or other untoward reaction when administered to an animal, or a human, as appropriate. Veterinary uses are equally included within the invention and "pharmaceutically acceptable" formulations include formulations for both clinical and/or veterinary use.

As used herein, "pharmaceutically acceptable carrier" includes any and all solvents, dispersion media, coatings, antibacterial, and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. For human administration, preparations should meet sterility, pyrogenicity, general safety and purity standards as required by FDA Office of Biologies standards. Supplementary active ingredients can also be incorporated into the compositions.

Examples of carriers include solvents and dispersion media containing, for example, water, ethanol, polyol (for example, glycerol, propylene glycol, and liquid polyethylene glycol, and the like), mixtures thereof, and vegetable oils. In many cases, it will be preferable to include isotonic agents, for example, by the use of a coating, such as lecithin, by the maintenance of the required particle size in the case of dispersion and/or by the use of surfactants.

The present disclosure contemplates the administration of the described pharmaceutical compositions by various routes. Pharmaceutical compositions comprising HDL, ApoA1, and/or ApoA1 mimetics of the invention may be administered by any route that ensures bioavailability in the circulation. These routes can include, but are by no means limited to parenteral administration, systemic administration, oral administration, nasal administration, rectal administration, intraperitoneal injection, intravascular injection, subcutaneous injection, transcutaneous administration, inhalation administration, and intramuscular injection.

Injectable preparations include sterile suspensions, solutions or emulsions of the active ingredient in aqueous or oily vehicles. The compositions may also contain formulating agents, such as suspending, stabilizing and/or dispersing agent. The formulations for injection may be presented in unit dosage form, *e.g.* in ampoules or in multidose containers, and may contain added preservatives.

Alternatively, the injectable formulation may be provided in powder form for reconstitution with a vehicle, including but not limited to sterile pyrogen free water, buffer, dextrose solution, etc., before use. To this end HDL, ApoA1 and/or ApoA1 mimetics of the present disclosure may be lyophilized, or the co-lyophilized peptide-lipid complex may be prepared. The stored preparations can be supplied in unit dosage forms and reconstituted prior to use in vivo.

For prolonged delivery, the active ingredient can be formulated as a depot preparation, for administration by implantation; *e*.*g.*, subcutaneous, intradermal, or intramuscular injection. Thus, for example, the active ingredient may be formulated with polymeric or hydrophobic materials (*e*.*g*., as an emulsion in an acceptable oil) or ion exchange resins, or as sparingly soluble derivatives; *e*.*g*., as a sparingly soluble salt form of the modified HDL, ApoA1 and/or ApoA1 mimetics.

Alternatively, transdermal delivery systems manufactured as an adhesive disc or patch which slowly releases the active ingredient for percutaneous absorption may be used. To this end, permeation enhancers may be used to facilitate transdermal penetration of the active ingredient.

For oral administration, the pharmaceutical compositions may take the form of, for example, tablets or capsules prepared by conventional means with pharmaceutically acceptable excipients, such as binding agents (*e*.*g*., pregelatinised maize starch, polyvinylpryrrolidone or hydroxypropyl methylcellulose); fillers (*e.g*., lactose, microcrystalline cellulose or calcium hydrogen phosphate); lubricants (*e.g.,* magnesium stearate, talc, or silica); disintegrants (*e.g*., potato starch or sodium starch glycolate); or wetting agents (*e*.*g*., sodium lauryl sulfate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives, such as suspending agents (*e*.*g*., sorbitol syrup, cellulose derivatives or hydrogenated edible fats); emulsifying agents (*e*.*g*., lecithin or acacia); non-aqueous vehicles (*e.g.,* almond oil, oily ester, ethyl alcohol or fractioned vegetable oils): and preservatives (*e.g.*, methyl or propyl-p-hydroxybenzoates or ascorbic acid). The preparations may also contain buffer salts, flavoring, coloring, and sweetening agents as appropriate. Preparations for oral administration may be suitably formulated to give controlled release of the active compound.

For buccal administration, the compositions may take the form of tablets or lozenges formulated in conventional manner. For rectal and vaginal routes of administration, the active ingredient may be formulated as solutions (for retention enemas) suppositories or ointments.

For administration by inhalation, the active ingredient can be conveniently delivered in the form of an aerosol spray presentation from pressurized packs or a nebulizer, with the use of a propellant, *e*.*g*., dichlorodifluoromethane, trichlorofluoromethane, dichlorotetrafluoroethane, carbon dioxide or other gas. In the case of a pressurized aerosol, the dosage unit may be determined by providing a valve to deliver a metered amount. Capsules and cartridges of e.g. gelatin for use in an inhaler or insufflators may be formulated containing a powder mix of the compound and a suitable powder base such as lactose or starch.

The compositions may, if desired, be presented in a pack or dispenser device, which may contain one or more unit of dosage forms containing the active ingredient. The pack may for example comprise metal or plastic foil, such as a blister pack. The pack or dispenser device may be accompanied by instructions for administration.

"Unit dosage" formulations are those containing a dose or sub-dose of the administered ingredient adapted for a particular timed delivery. For example, exemplary "unit dosage" formulations are those containing a daily dose or unit or daily sub-dose or a weekly dose or unit or weekly sub-dose and the like.

Under ordinary conditions of storage and use, all such preparations should contain a preservative to prevent the growth of microorganisms. The prevention of the action of microorganisms can be brought about by various antibacterial and antifungal agents, for example, parabens, chlorobutanol, phenol, sorbic acid, thimerosal, and the like. Prolonged absorption of the injectable compositions can be brought about by the use in the compositions of agents delaying absorption, for example, aluminum monostearate and gelatin.

Prior to or upon formulation, HDL, ApoA1 and/or ApoA1 mimetics should be extensively dialyzed to remove undesired small molecular weight molecules, and/or lyophilized for more ready formulation into a desired vehicle, where appropriate. Sterile injectable solutions are prepared by incorporating the active ingredients in the required amount in the appropriate solvent with various of the other ingredients enumerated above, as desired, followed by filtered sterilization. Generally, dispersions are prepared by incorporating the various sterilized active ingredients into a sterile vehicle that contains the basic dispersion medium and the required other ingredients from those enumerated above.

In the case of sterile powders for the preparation of sterile injectable solutions, the preferred methods of preparation are vacuum-drying and freeze-drying techniques that yield a powder of the active ingredient, plus any additional desired ingredient from a previously sterile-filtered solution thereof.

Pharmaceutical "slow release" capsules or "sustained release" compositions or preparations may be used and are generally applicable. Slow release formulations are generally designed to give a constant drug level over an extended period and may be used to deliver ApoA1 mimetic polypeptides or fragments thereof in accordance with the present disclosure. In certain examples, liposomes and/or nanoparticles may also be employed with the HDL, ApoA1 and/or ApoA1 mimetics. The formation and use of liposomes is generally known to those of skill in the art, as summarized below. Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 Å, containing an aqueous solution in the core. HDL, ApoA1 and/or ApoA1 mimetics can also formulated be into phospholipid discs of between 8 and 20 nm, through spontaneous reaction with phospholipid liposomes, or through the cholate dialysis procedure.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present disclosure, and such particles may be are easily made.

In a further aspect of the present disclosure, HDL, ApoA1 and/or ApoA1 mimetics can be used in combination and adjunctive therapies for inhibiting cancer cell proliferation and growth. The phrase "combination therapy" embraces the administration of HDL, ApoA1 and/or ApoA1 mimetics and an additional therapeutic agent as part of a specific treatment regimen intended to provide a beneficial effect from the co-action of these therapeutic agents. Administration of these therapeutic agents in combination typically is carried out over a defined time period (usually minutes, hours, days or weeks depending upon the combination selected). The phrase "adjunctive therapy" encompasses treatment of a subject with agents that reduce or avoid side effects associated with the combination therapy of the present disclosure.

A combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by an appropriate route including, but not limited to, oral routes, intravenous routes, intramuscular routes, and direct absorption through mucous membrane tissues. There therapeutic agents can be administered by the same route or by different routes. The sequence in which the therapeutic agents are administered is not narrowly critical.

Combination therapy also can embrace the administration of the therapeutic agents as described above in further combination with other biologically active ingredients (such as, but not limited to, a second and different therapeutic agent) and non-drug therapies (such as, but not limited to, surgery or radiation treatment). Where the combination therapy further comprises radiation treatment, the radiation treatment may be conducted at any suitable time so long as a beneficial effect from the co-action of the combination of the therapeutic agents and radiation treatment is achieved. For example, in appropriate cases, the beneficial effect is still achieved when the radiation treatment is temporarily removed from the administration of the therapeutic agents, perhaps by days or even weeks.

In certain examples, HDL, ApoA1 and/or ApoA1 mimetics can be administered in combination with at least one anti-proliferative agent selected from the group consisting of chemotherapeutic agent, an antimetabolite, and antitumorgenic agent, and antimitotic agent, and antiviral agent, and antineoplastic agent, an immunotherapeutic agent, and a radiotherapeutic agent.

The phrase "anti-proliferative agent" can included agents that exert antineoplastic, chemotherapeutic, antiviral, antimitotic, antitumorgenic, and/or immunotherapeutic effects, e.g., prevent the development, maturation, or spread of neoplastic cells, directly on the tumor cell, e.g., by cytostatic or cytocidal effects, and not indirectly through mechanisms such as biological response modification. There are large numbers of anti-proliferative agent agents available in commercial use, in clinical evaluation and in pre-clinical development, which could be included in the present invention by combination drug chemotherapy. For convenience of discussion, anti-proliferative agents are classified into the following classes, subtypes and species: ACE inhibitors, alkylating agents, angiogenesis inhibitors, angiostatin, anthracyclines/DNA intercalators, anti-cancer antibiotics or antibiotic-type agents, antimetabolites, antimetastatic compounds, asparaginases, bisphosphonates, cGMP phosphodiesterase inhibitors, calcium carbonate, cyclooxygenase-2 inhibitors, DHA derivatives, DNA topoisomerase, endostatin, epipodophylotoxins, genistein, hormonal anticancer agents, hydrophilic bile acids (URSO), immunomodulators or immunological agents, integrin antagonists, interferon antagonists or agents, MMP inhibitors, miscellaneous antineoplastic agents, monoclonal antibodies, nitrosoureas, NSAIDs, ornithine decarboxylase inhibitors, pBATTs, radio/chemo sensitizers/protectors, retinoids, selective inhibitors of proliferation and migration of endothelia cells, selenium, stromelysin inhibitors, taxanes, vaccines, and vinca alkaloids.
The major categories that some anti-proliferative agents fall into include antimetabolite agents, alkylating agents, antibiotic-type agents, hormonal anticancer agents, immunological agents, interferon-type agents, and a category of miscellaneous antineoplastic agents. Some anti-proliferative agents operate through multiple or unknown mechanisms and can thus be classified into more than one category.

Another aspect of the present disclosure relates to a method of treating cancer in a subject by administering to the subject at least one agent that stimulates the cellular receptors for HDL, the SR-B1 receptor, and/or the cellular cholesterol transporters ABCA1 and ABCG1. The agent can comprise at least one of small molecules, peptide mimetics, antibodies or fragments thereof and be administered to the subject to protect the subject against a wide variety of cancers including but not limited to malignant melanoma, lung, breast, colon, brain, kidney, bladder, prostate, pancreatic, oral, head and neck, sarcomas, lymphomas (B-cell and T-cell) and adrenal cancer development as well as slow development of tumors, slow tumor metastasis, and lengthen survival time of the subject.

A further aspect of the present disclosure relates to a diagnostic method for characterizing a subject's risk for cancer. The diagnostic method can included determining the level of dysfunctional HDL in a subject and comparing the measured level to a control value. An increase in the measured level of dysfunctional HDL compared to the control value can correlate with lack of protection against a wide variety of cancers including, malignant melanoma, lung, breast, colon, brain, kidney, bladder, prostate, pancreatic, oral, head and neck, sarcomas, lymphomas (B-cell and T-cell) and adrenal cancer development; facilitate development of tumors, tumor metastasis and shorten survival times.

The following examples are included to demonstrate preferred examples of the present disclosure. It should be appreciated by those of skill in the art that the techniques disclosed in the examples which follow represent techniques discovered by the inventor to function well in the practice.

### EXAMPLES

### EXAMPLE 1

### ApoA1 and DHDL acts to retard cancer growth, development and metastasis and activates a subset of innate immune host defense genes and decreases a subset of cellular proliferation genes and genes associated with cancer.

HDL isolated from human plasma was used initially to treat bone marrow-derived macrophages grown in culture from wild type (WT) C57/BL6 mice at various concentrations ranging from non to high physiological levels (750 microgram protein/ml). Cells were harvested after 8 hour exposure to HDL and total RNA prepared and used for gene chip analysis. HDL exposure resulted in increased expression of a number of genes associated with innate immune functions in addition to those associated with cholesterol metabolism. Genes that were down-regulated by HDL exposure included a subset of genes associated with cellular proliferation and genes associated with cancer progression and metastasis. These finding led to the hypothesis that HDL and perhaps ApoA1 its major protein constituent may play an important role in protection from cancer or tumorogenesis.

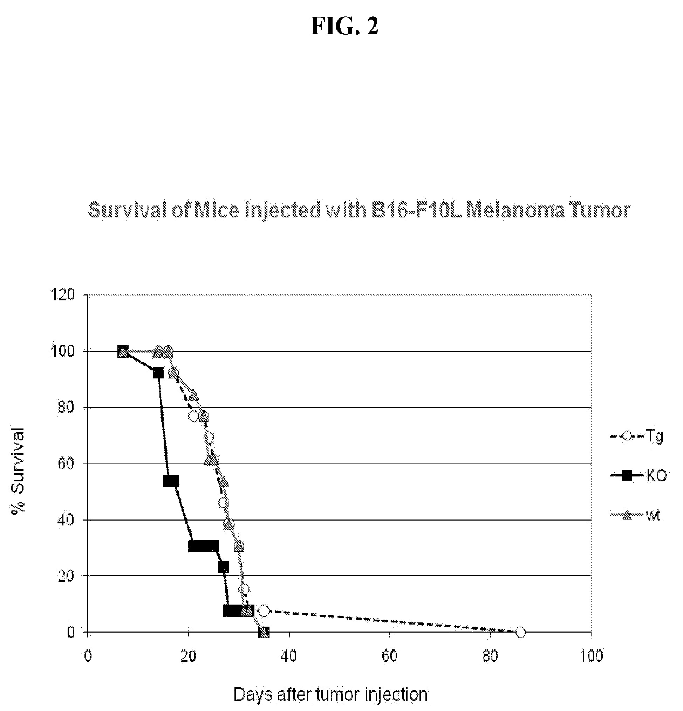

To test this hypothesis, the behavior of a metastatic murine melanoma cell line, B16F10L, was examined after inoculating 100,000 cells per site on the left and right dorsal flank of ApoA1 deficient, wild type or human ApoA1 expressing transgenic mice. Figs. 1-3 show that ApoA1 deficient mice (ApoA1-/-) are more susceptible to malignant melanoma development, tumor burden and survival time than wild type C57/BL6 (WT) mice littermate mice or to human ApoA1 expressing transgenic (ApoA1-Tg)/C57/BL6 mice. The ApoA1-Tg mice are more protected from malignant melanoma development, tumor burden and have a longer survival time than the WT mice. Total tumor burden is lowest in ApoA1 transgenic animals as was metastasis as determined by ventral side imaging of tumors. ApoA1 is the major protein component of high density lipoprotein (HDL). HDL plasma levels were highest in the ApoA1-Tg mice followed by the WT mice and the ApoA1 deficient mice were devoid of HDL as measured.

It was hypothesized that ApoA1 is a key regulator of this anti-cancer protective response. Peptide mimetics of HDL have been reported and those peptides enhance the anti-inflammatory function of HDL and promote enhanced cholesterol efflux in vivo. It is anticipated that these mimetics and others that provirle enhanced HDT function will also prove to have an anti-cancer protective response. It also follows that oxidant resistant forms of apoA1 that can be reconstituted into nascent HDL and shown to provide enhanced HDL function under conditions that result in oxidatively damaged HDL (and loss of anti-inflammatory, and cholesterol efflux activity) will provide even greater protection than wild type apoAI when infused.

There are a number of different oxidant resistant forms of apoAI that include residues that when mutated from tryptophan's to phenylalanines provide protection from loss of cholesterol efflux activity 4WF Apoa1 and 72F ApoA1. Tyrosine 166 mutated to glutamic acid (E) or aspartic acid (D) protects nascent HDLs reconstituted with this version of ApoA1 to interact and stimulate Lecithin-cholesterol acyltransferase (LCAT) binding and are capable of stimulating LCAT activity. Another oxidant resistant form of ApoA1 will have residues different from those described above that when mutated fail to activate a proinflammatory cellular reaction including activation of nuclear factor kappa B transcription factor, or vascular cell adhesion molecule (VCAM) expression. Combinations of all of these various mutants alone and in combination with each other and the resulting ApoA1 expressed from them is expected to provide an enhanced anti-cancer protective response when used alone or in various phospholipid mixtures or in various phospholipid/cholesterol reconstituted nascent HDL particles.

It is also possible that once the most potent version of ApoA1 has been identified that has anti-cancer protective properties it could be used, for example, in *in vivo* gene therapy by targeting the mutant allele for expression in the tumor vasculature using peptide display along with cell-type specific promoters to drive ApoA1 expression primarily in the tumor vasculature.

HDL when oxidized becomes dysfunctional and loses its anti-inflammatory properties and also its ability to promote cholesterol efflux and to bind its receptor scavenger receptor B 1 (SR-B1) and the cellular cholesterol transporters ABCA1 and ABCG1. Athelerosclerotic appearing vasculature has been noted in the tumor beds of a number of cancers cited in the literature. It is anticipated that increased levels of dysfunctional HDL negatively impact HDL's ability to provide protection from cancer. Accurate measurement of levels of dysfunctional HDL should give a predictive measurement of how well an individual is protected by identifying the true levels of protective HDL.

### EXAMPLE 2

### HDL exposure to macrophages induces gene expression from innate immune function genes and down regulates expression of cellular proliferation genes and genes associated with cancer progression and metastasis.

Bone marrow-derived macrophages from wild type female C57B1/6 mice (10-11 wks old) were treated with increasing concentration of reconstituted HDL (up to 750 mg/ml) made with human plasma-purified ApoA1). Total RNA was isolated after 7 hours treatment and processed for gene chip microarray studies using Illumina's mouse Ref-8 8 v1.1 beadchip. Expression intensities were normalized and background corrected using Beadstudio software. A 2-fold difference in expression with respect to control (PBS) treatment and a detection p-value <0.01 was taken as being significant.

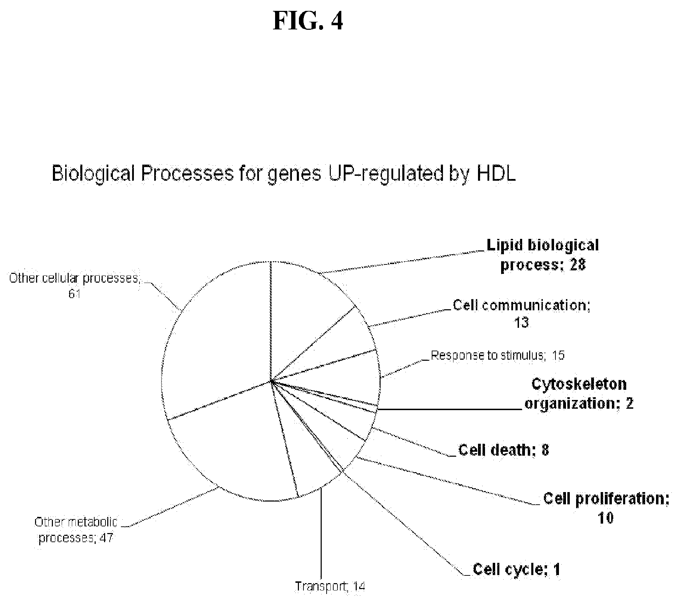

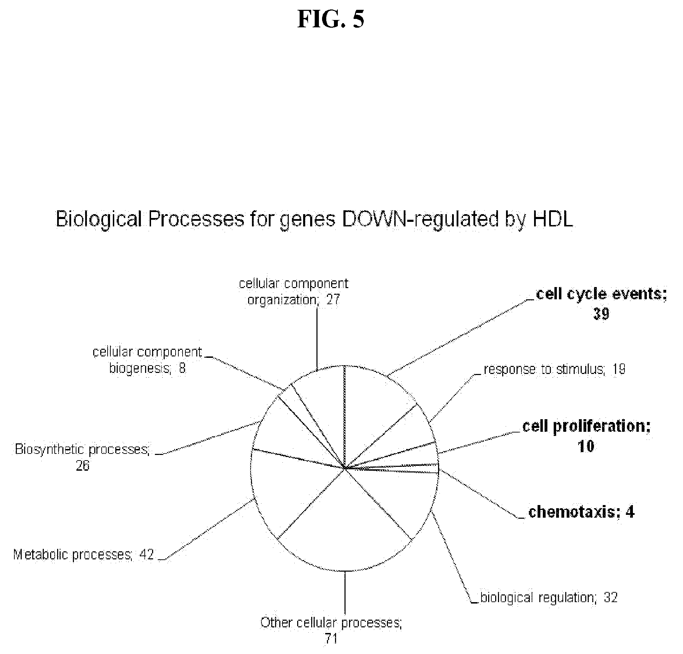

Using the aforementioned criteria from two independent experiments, 99 and 269 genes were found that were up and down regulated by HDL, respectively. Gene chip data was validated by TaqMan real time RT PCR on a whole panel of genes in these studies. As illustrated schematically in Figs. 4 and 5, ingenuity bioinformatics software was used to examine the biological pathways enriched by the genes regulated by HDL in these experiments. Some of the up regulated genes (28 of 99) mapped primarily to lipid biological processes that included cholesterol biosynthesis and transport. Significantly, 10 up-regulated genes have anti-proliferative function. (Fig. 4) The top biological function for down-regulated genes was the cell cycle, with the regulated genes having primarily pro-proliferation activities. (Fig. 5) Additionally, genes that positively regulate tumor development, tumor metastasis and immune cell trafficking were amongst those down-regulated by HDL.

### EXAMPLE 3

### ApoA1 Inhibits Tumor Formation

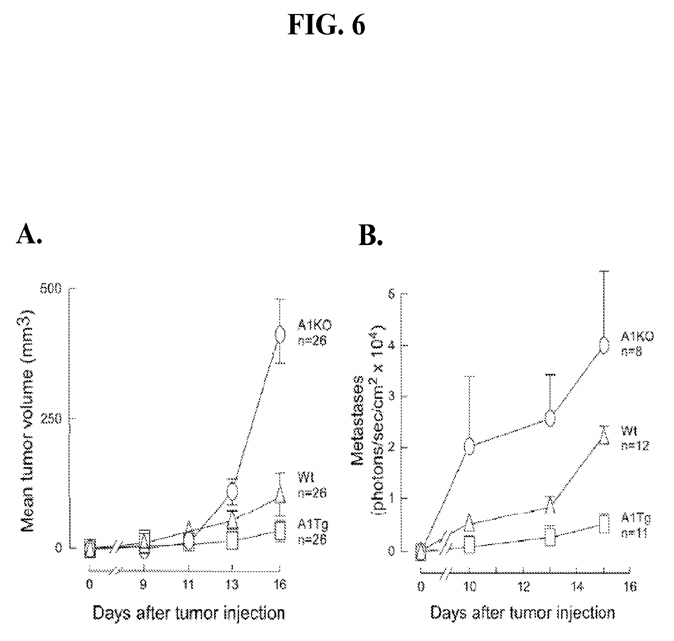

In this the first part of this Example, Tg mice that were injected with F10L_B16 melanoma tumors were given gene therapy with a vector expressing apoA1. It was found that gene therapy with apoA1 is protective for cancer development (tumor growth and metastases formation). ApoA1 transgenic mice show reduced tumor burden (Figure 6A), growth rate (Figure 6A), and metastases (Figure 6B) formation relative to wild type (WT) mice. A1KO (apoA1 knock out) mice were also injected with F10LB16 melanoma cells and tumor development was monitored. The results indicated that genetic deletion of apoA1 (apoA1-KO mice) are more susceptible to development of tumor (Figure 6A), growth rate of tumor (Figure 6A), and metastases formation (Figure 6B). In this Example, abbreviations are as follows: KO= ApoA1 null on C57B1/6 background, tg= human ApoA1 expressing transgenic C57B1/6, Wt= wild type C57B1/6.

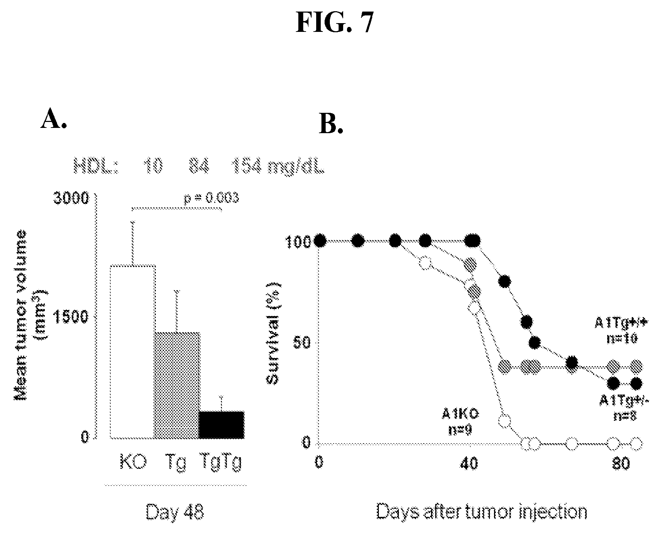

In another part of this Example, KO and Tg or Tg/Tg mice were injected with Lewis lung cancer cells and monitored for tumor formation. Figure 7A shows the mean tumor volume in the mice after 48 days, and Figure 7B shows the survival percentage over time. The Tg mice had smaller tumor sizes and lived longer than the KO mice, showing the protective effect of ApoA1.

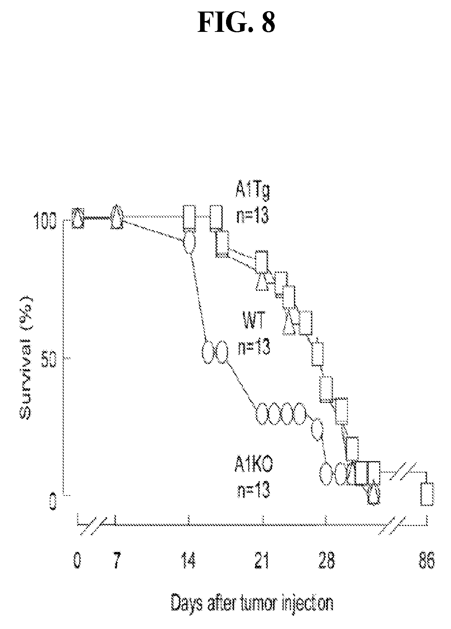

In another part of this Example, Tg, WT, and A1KO mice were injected with F10L_B16 melanoma tumor cells and monitored for survival. The results are shown in Figure 8.

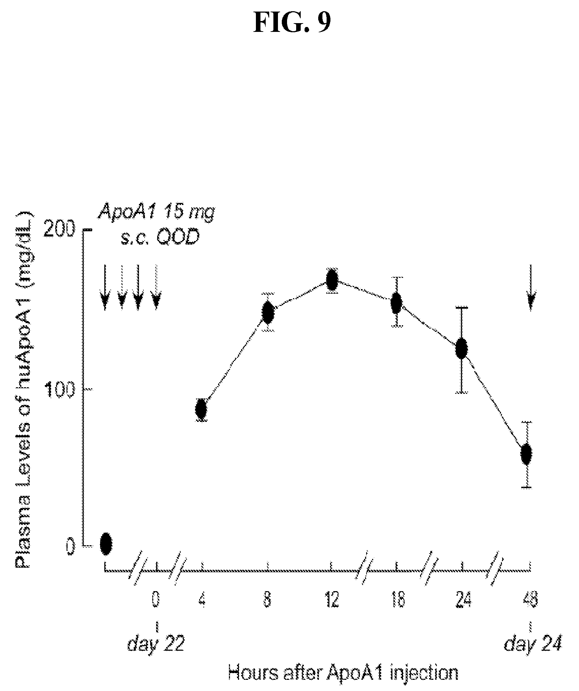

In another part of this Example, apoA1 was injected subcutaneously into mice every other day and the plasma levels of apoA1 at steady state (shown in Figure 9 for says 22-24) were monitored. Figure 9 shows that the plasma level of apoA1 is between 75-175 mg/dL. This approximates the plasma levels of apoA1 in humans.

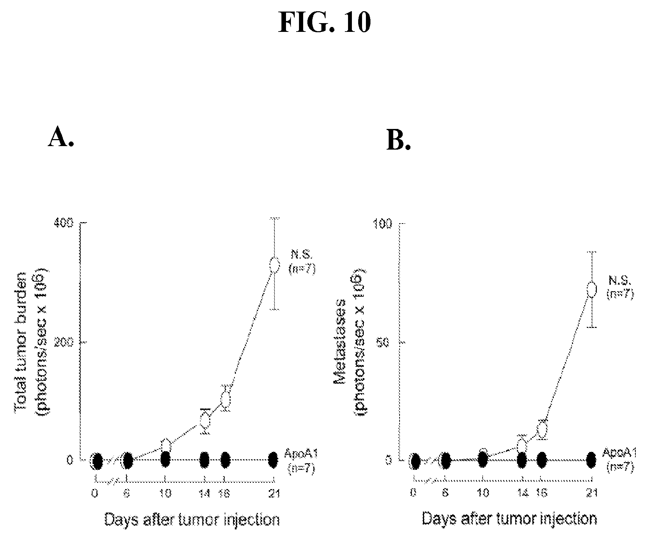

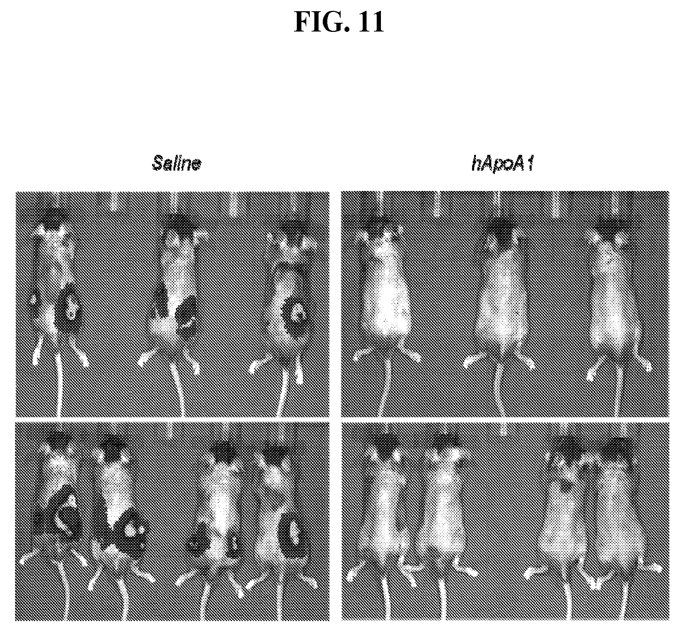

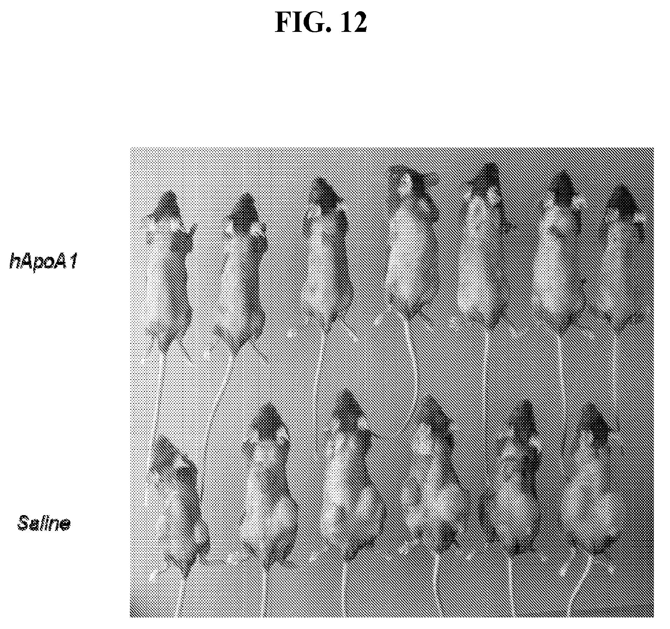

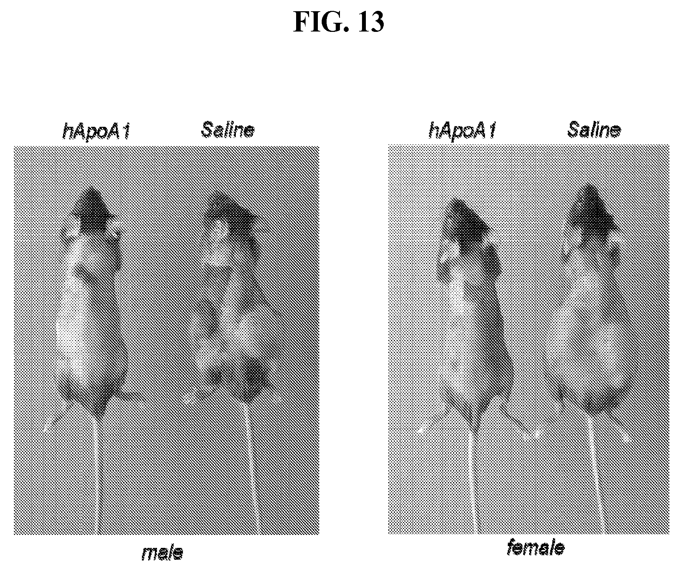

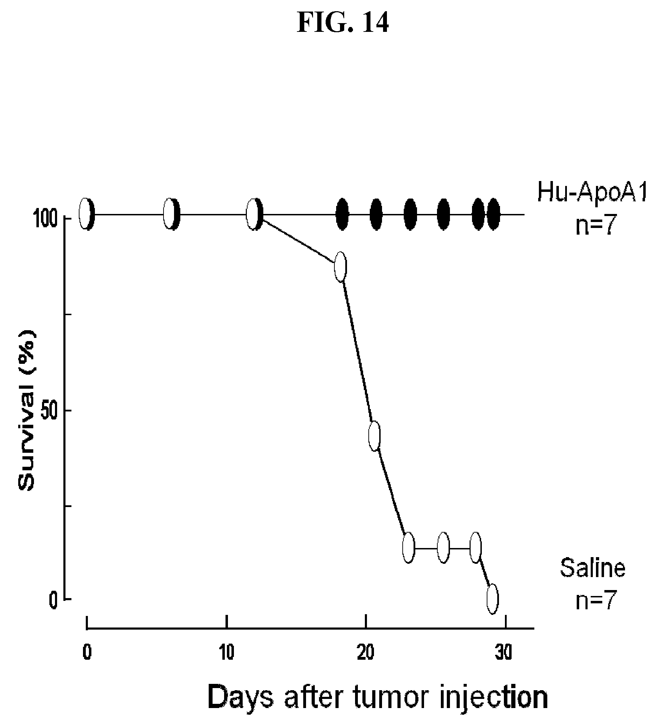

In another part of this Example, human ApoA1 or normal saline (control) was given to apoA1-KO mice throughout duration of the study following the dosing schedule of 15 mg s.c. QOD. 10⁵ F10L_B16 Melanoma Tumor cells were injected subcutaneously into the dorsum of the mice. Total tumor burden and metastases were quantified by xenogen imaging. The results for total tumor burden are shown in Figure 10A and the results for metastases are shown in Figure 10B. This data shows that giving apoA1 to mice blocks tumor growth and metastases formation. Images of the mice 16 days post tumor injection are shown in Figure 11, which show much greater tumor growth in the saline treated mice. Images of the mice 21 days post tumor injection are shown in Figure 12, which show much greater tumor growth in the saline treated mice. A comparison by gender of the mice from Figure 12 is shown in Figure 13. The survival for these mice was also monitored and the results are shown in Figure 14, which shows that the ApoA1 treated mice had much greater survival.

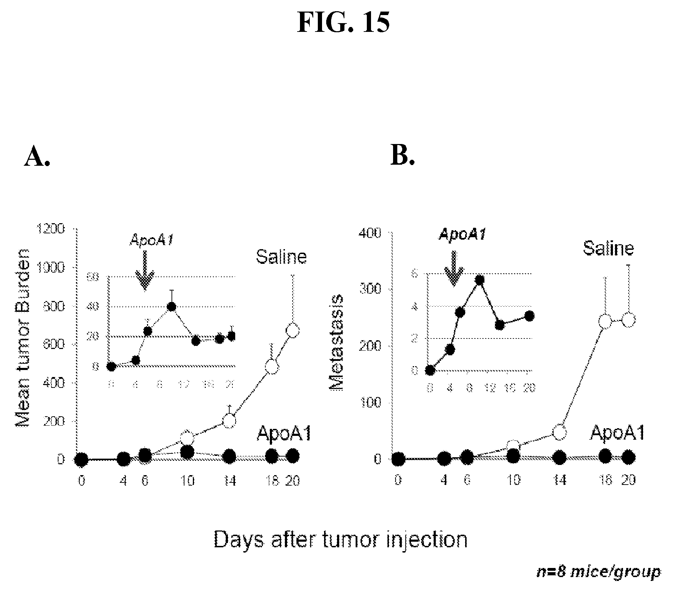

In another part of this Example, the impact of giving apoA1 as a therapy is evaluated after the tumor growth is established and metastases are detectable. The same malignant melanoma animal model is employed. ApoA1 injections were started one week after tumor injection. Overall tumor growth (Fig. 15a), and metastases formation (Fig. 15B), are dramatically reduced in the animals that receive apoA1. In the insets for both 16A and 16B, note that apoA1 injections actually shrink peak tumor volume by ∼50% within 1-2 weeks of therapy. A similar ∼50% reduction in peak metastases burden is also noted in the apoA1 treated mice. These results indicate that apoA1 treatment in established cancer can not only halt progression of tumor and metastases growth, but can actually reduce the extent of tumor burden, and shrink metastases.

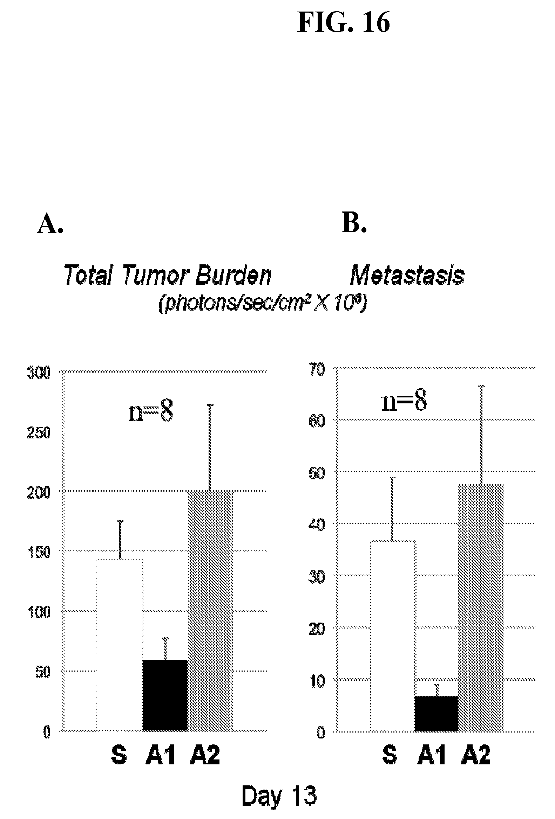

In similar experiments, ApoA2 was given in the same dosing strategy as apoA1 vs normal saline (S) control. As shown in Figure 16, ApoA1 is markedly more potent at reducing cancer growth (Fig. 16A) and metastases formation (Fig. 16B).

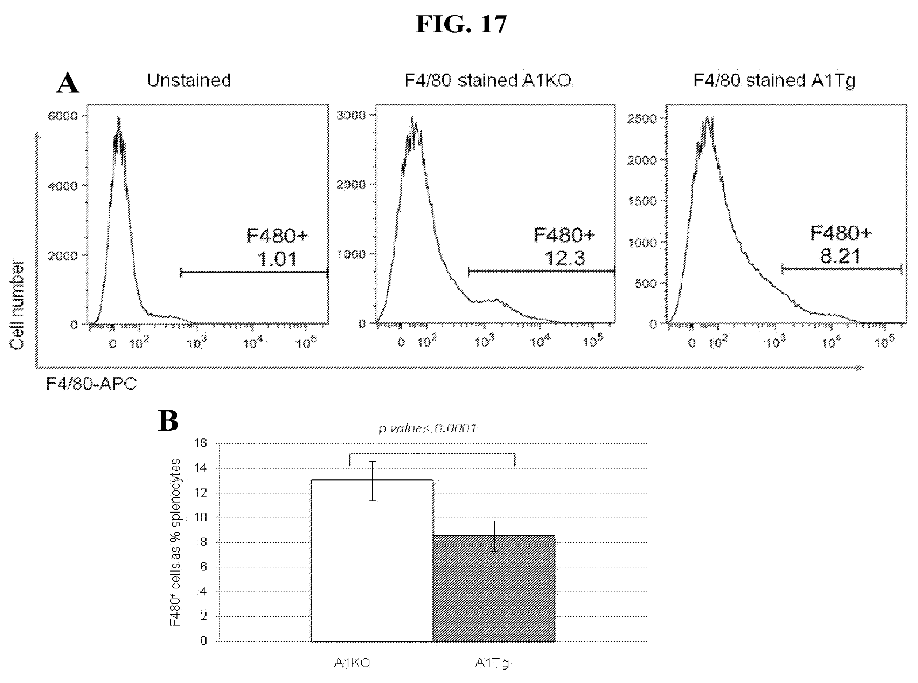

In another part of this Example, spleen macrophages from B16_F10L tumor-bearing A1KO mice and A1Tg mice were compared. Spleen macrophages were quantified by flow cytometry as F4/80+ positive cells. The results are shown in Figure 17, including cell number (Fig. 17A) and F480+ cells as a percentage of splenocytes (Fig. 17B.). While the present invention is not limited to any particular mechanism and an understanding of the mechanism is not necessary to practice the invention, these results suggest that one mechanism that apoA1 helps to reduce cancer development is through immunomodulatory effects on innate immune cells. This data shows that apoA1 treatment lowers tumor associated macrophage numbers.

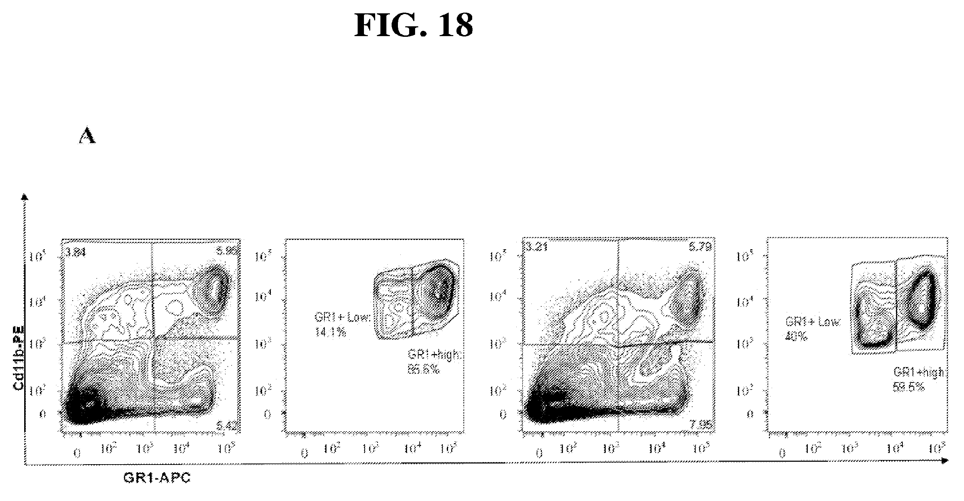

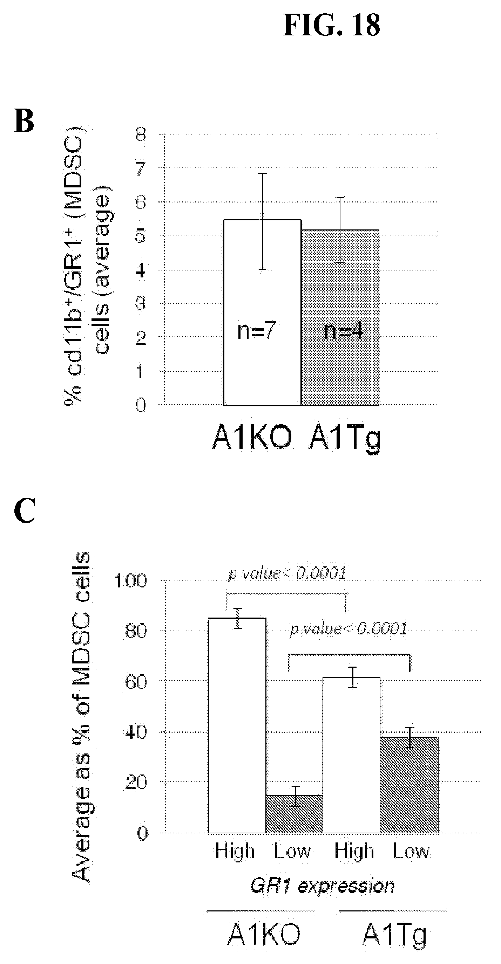

In another part of this Example, myeloid derived suppressor cells (MDSCs) from the spleens of fB16_F10L tumor-bearing A1KO mice and A1Tg mice were compared. The results are shown in Figure 18. Figure 18A shows a cell plot of Cd11b-PE vs. GR1-APC for AIKo and A1 Tg derived cells. Figure 18B the average percentage of cd1b+/GR1+ (MDSC) cells for A1KO and A1Tg derived cells. Figure 18C shows the average as a percentage of MDSC cells for A1KO and A1Tg cells. Myeloid derived suppressor cells (MDSCs) play a major role in combating cancer growth and development. The data in Figure 18 shows that apoA1 alters the phenotype of MDSCs into those that are associated with better cancer fighting capabilities, and improved overall outcomes. MDSCs are heterogeneous, made in bone marrow and recruited to tumor site and other organs such as spleen. High GR1+/CD11b+ MDSCs are neutrophilic (help tumors by expressing Mmp9, S100a8, S100a9, etc). Low GR1+ / CD11b+ expressing MDScs are more monocytic (not as pro-tumorigenic as neutrophils). In many tumors % of high GR1+ (neutrophilic) correlates with tumor progression. ApoA1 treatment (either direct injection, or via gene therapy as in this case with transgenic mice) reduces the high GR1+ MDSC level. While the present invention is not limited to any particular mechanism and an understanding of the mechanism is not necessary to practice the invention, these results further suggest that one mechanism that apoA1 helps to reduce cancer development is through immunomodulatory effects on innate immune cells.

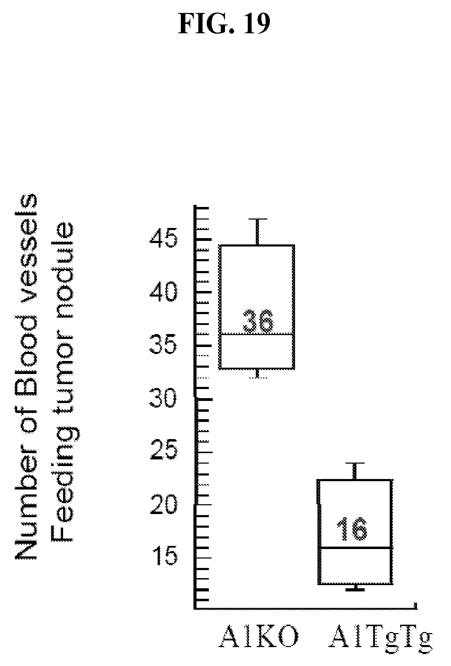

In another part of this Example, the effect of apoA1 on angiogenisis was measured (results shown in Figure 19). This data shows that apoA1 is anti-angiogenic, another potential mechanism through which it may promote its anti-cancer and anti-metastases function (although the present invention is not limited to any particular mechanism and an understanding of the mechanism is not necessary to practice the invention). Tumors can only grow so much when fed by diffusion from an adjacent capillary. Beyond a certain size, they need to have new blood vessels grow into them to feed the expanding nucleus of cells. Metastases similarly need new vessel growth to develop. In this Example, malignant melanoma cancer cells were injected into apoA1 KO and TG mice. At approximately 1 week of age, tumors were excised and the numbers of vessels feeding the tumor cells directly quantified by microscopy. Note that the numbers of vessels is markedly reduced in the mice with appA1.

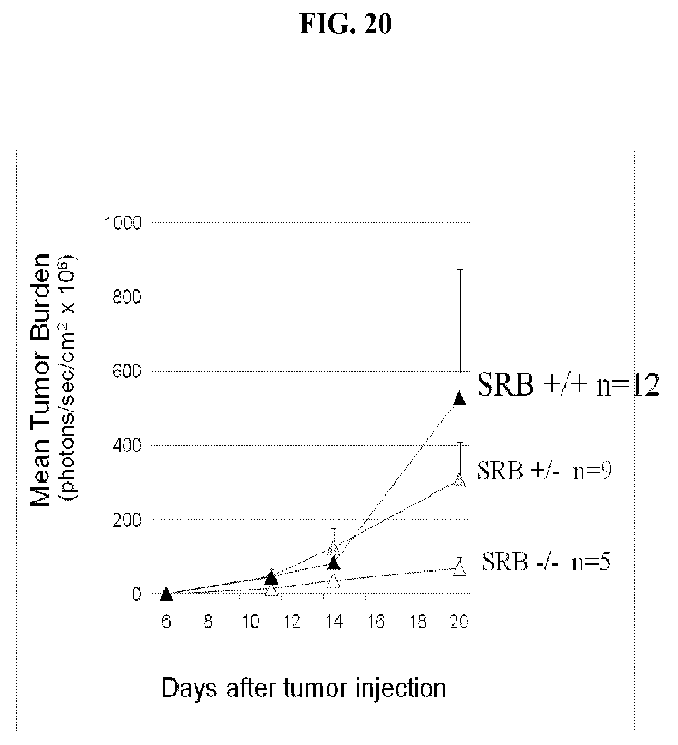

In another part of this Example, work was conducted that examined the role of SRB1, the classic HDL receptor, in the anti-cancer activities of apoA1 and HDL. Mice that lacked SRB1 (both heterozygous and homozygous) were injected with melanoma cells (B16F10L-luciferase). As shown in Figure 20, animals that lack SRB1 (both heterozygotes and homozygotes) showed protection from cancer growth, while the SRB1 containing mice (SRB1+/+) have a greater rate of tumor growth. While the present invention is not limited to any mechanism, these results appear to show two things. First, SRB1, the classic HDL receptor, is not playing a major protective role in cancer mediated by apoA1 Second, since mice lacking SRB1 have higher apoA1 and HDL particle levels, these results strongly suggest that a HDL receptor alternative to SRB1 mediates the beneficial effects of apoA1 and HDL on tumor growth and metastases.

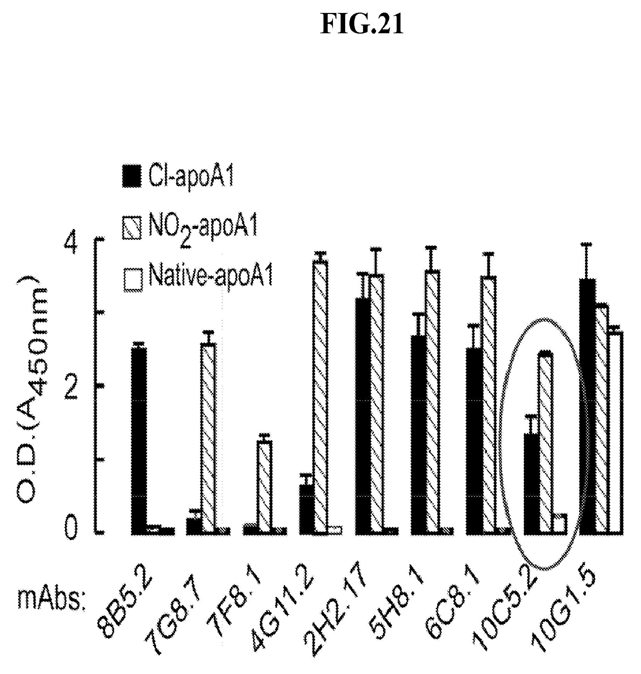

In other work, various monoclonal antibodies that target different forms of oxapoA1 were used to screen malignant melanoma human cancer biopsy specimens. The results are shown in Figure 21.

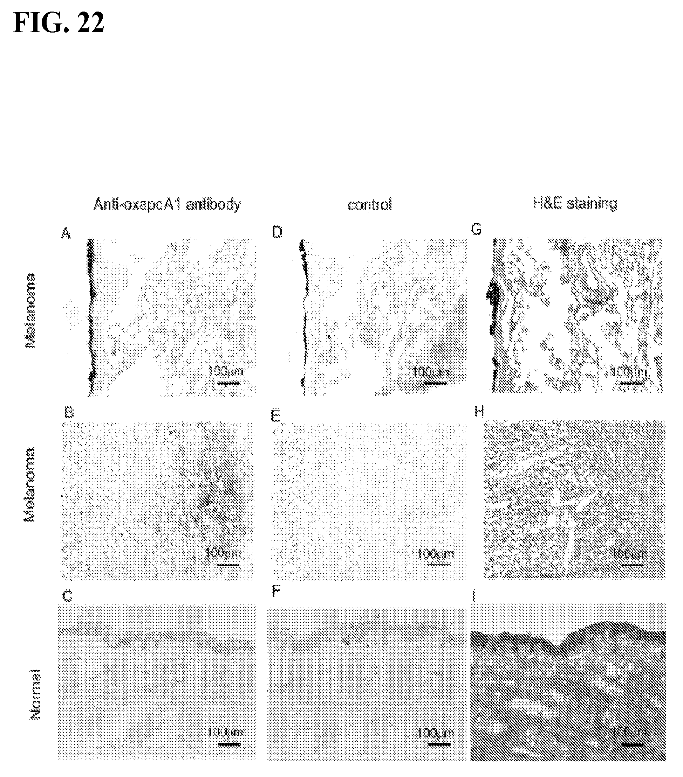

In other work, immunohistochemistry on the human melanoma skin and normal skin tissue was carried out on fresh frozen sections. The anti-human ox-apoA1 mouse monoclonal antibody 10C5.2 was used at 10 ug/ml. Results are shown in Figure 22, where A-C show ox-apoA1 staining; D-F show mouse IgG1 control staining, and G-I show H&E staining. These results showed that antibodies specific to oxidized apoA1 intensely stain human malignant melanoma tumor but not normal biopsy.

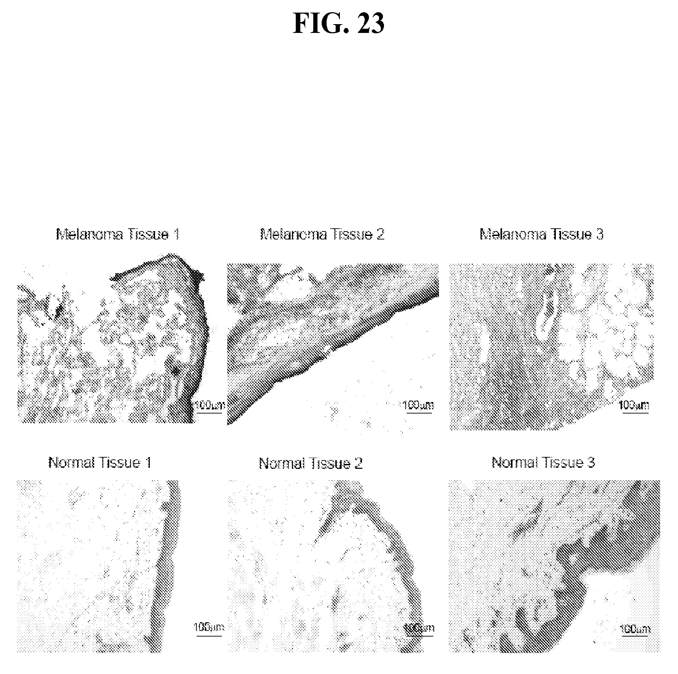

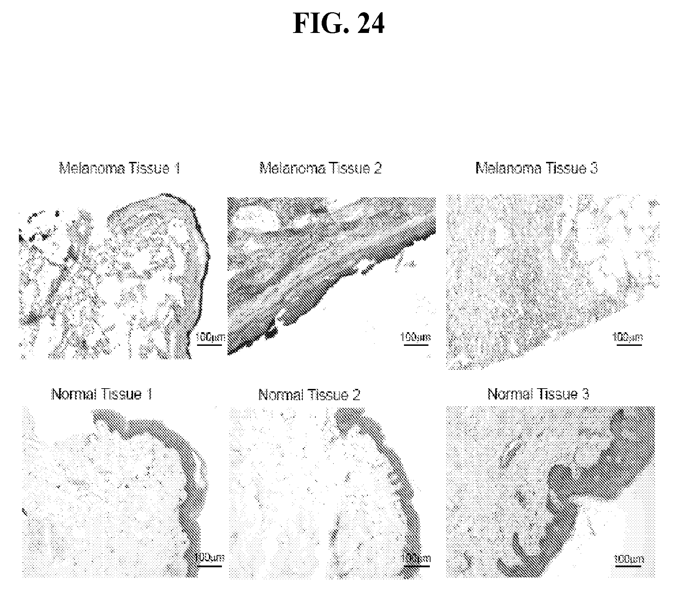

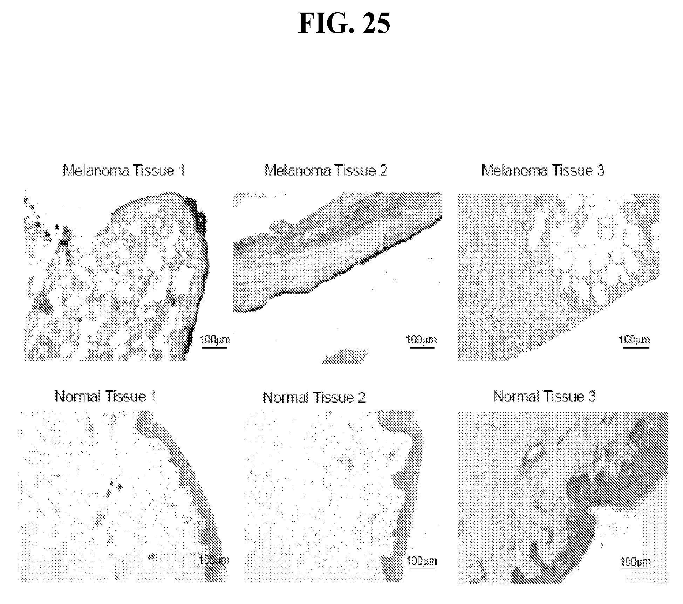

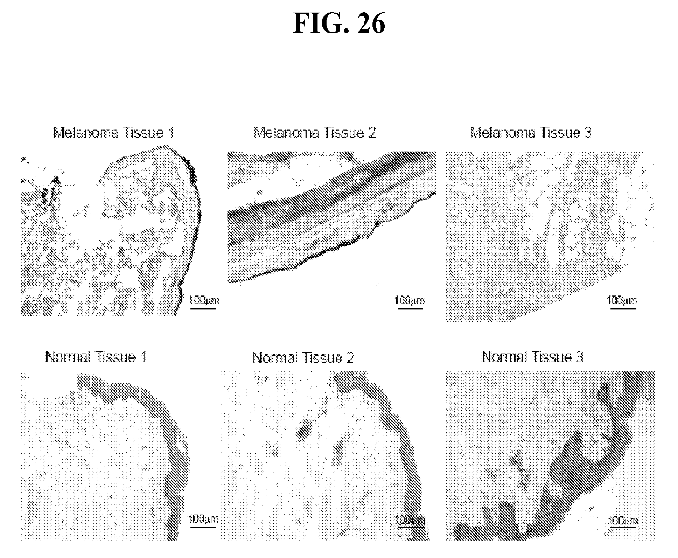

In other work, three mAbs shown to be specific for oxidized apoA1 were used to stain human malignant melanoma tumors. The oxidized apoA1 specific antibodies were each shown to intensely stains multiple human malignant melanoma tumors but not normal biopsy. Figure 23 shows the results for Mab 10G1.5, Figure 24 shows the results for Mab 10C5.2, Figure 25 shows the results for Mab 4611.2, while Figure 26 shows the results of a Mab control antibody.

### EXAMPLE 4

### ApoA1 Inhibits Tumor Formation

The example provides further evidence that ApoA1 inhibits tumor formation.

Mice deficient (SRB-1-/-) or heterozygous (SRB-1+/-) for HDL receptor, SRB-1, were injected with B16F10L-luciferase (1x105 cells s.c., per flank) and tumor growth was monitored by IVIS Xenogen live imaging (A, n= number of mice). Results are shown in Figure 27A. Figure 27B shows stimulation of allogenic T cell proliferation by splenic dendritic cells (DCs) isolated from naive or B 16F 10L-luciferase tumor bearing mice of indicated genotypes (n= number of mice) 14 days post tumor cell injection. Data in Figure 27C, where n= number of tumor injection sites, shows the terminal (day 21 post tumor injection; s.c. bilaterally) tumor volume measurements in B16F10L-luciferase bearing NSG mice injected (s.c., away from the site of tumor injection) every other day starting on day 1 post tumor injection with ApoA1 (20 mg/animal) or normal saline. Flow cytometry was performed on splenocytes from mice (n=number of animals) injected with normal saline (naive), B16F10L-luciferase (1x105 cells s.c., bilaterally) (27D) or injected with B16F10L-luciferase cells at 4 separate sites (1x105 cells s.c per site) on the dorsal side (27E & F)) harvested on day 14 (D) or 12 (E & F) post tumor injection. Data points are mean +/-SEM.

The results in Figure 27 show: (i) The classic HDL receptor, SRB1, is not involved in the therapeutic response from apoA1, since the absence of SRB1 is protective. This is consistent with elevated apoA1 and HDLc levels in the SRB1 null mouse, and the apoA1 acting perhaps via an alternative unknown receptor; (ii) The beneficial effect of apoA1 is not mediated by changes in T cell proliferation by dendritic cells; (iii) Adaptive immunity, which is impaired in the NSG mice (lacking T cells, B cells and NK killer cells), is not involved in the protective actions of apoA1 in cancer; (iv) In the absence of apoA1, there is an increase in CD11b+Ly6C+, and CD11bGR1+ high positive splenocytes, all indicative of adverse clinical outcomes. Conversely, genetic augmentation of apoA1 levels (transgenic mouse) improves these adverse markers; and (v) There is a reduction in the number of tumor associated macrophages (as monitored by F480+ antigen by flow cytometry) promoted by genetic enhancement of apoA1 production.

Angiogenesis was assessed in B16F10L melanoma bearing mice 7 days post tumor injection (s.c.,1x105 cells per flank). Tumor photographs were subjected to VESGEN analysis whereby color photomicrographs (Figure 28A) were converted to input vascular binary images (Figure 28B). The region of interest (ROI) representing the tumor mass (white in B) defined the perimeter of the tumor. The output of VESGEN was a series of color Generation Maps (colored vessels on black background) in which the largest diameter vessels were defined as G1 (red), with each subsequent smaller generation represented as G2 - G6 (C). Data in Figures 28D, E, and F were generated from panel C and show number of blood vessels based on vessel diameter (D), total vessel area (E) and vessel length density (F). (G) Number of vessels feeding directly into the primary tumor was counted under a microscope where n= tumor injection sites.

The data in Figure 28 collectively show that apoA1 promotes an anti-angiogenic (decreased number, cross sectional area, and length density) effect in cancer model. This biological effect is a mechanism which likely contributes to the observed anti-tumor and anti-metastases activity of apoA1 therapy.

Primary subcutaneous B16F10L tumors were harvested from A1KO and A1Tg tumor bearing mice (8 animals per genotype) 7 days post injection (s.c.,1x105 cells per flank). Total tumor protein extracts were prepared and subjected to Western blotting. Specific protein bands were detected with Odyssey infrared imaging system with actin serving as loading control. Results are shown in Figure 29A. Figure 29B shows MMP-9 activity in tumor extracts (in A) was assayed using Quickzyme assay kit according to the manufacturer's protocol. Pro-MMP-9 refers to total activity observed following chemical activation (APMA). Total activity includes the active enzyme (active MMP-9).

The data in Figure 29 shows that the amount and activity of MMP-9, a matrix metalloprotease that is critical for tumor invasion and metasteses development, is reduced by apoA1 (higher in the apoA1-KO and reduced in the apoA1 transgenic mouse). This data also shows that Calprotectin (S100A8/A9), an abundant protein in neoplastic tumor cells and believed to both serve as an adverse cancer marker and to promote inflammation-associated cancer, is markedly reduced by apoA1 (higher in the apoA1-KO and reduced in the apoA1 transgenic mouse).

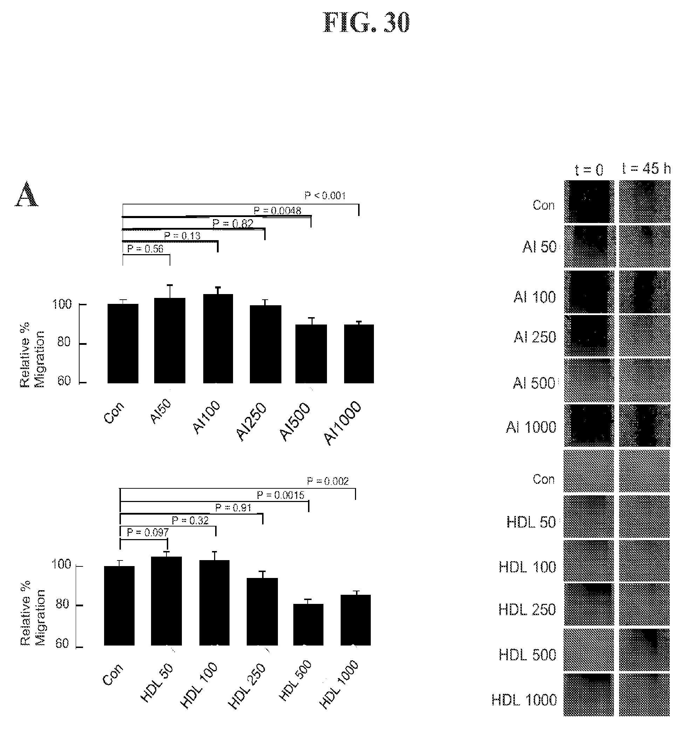

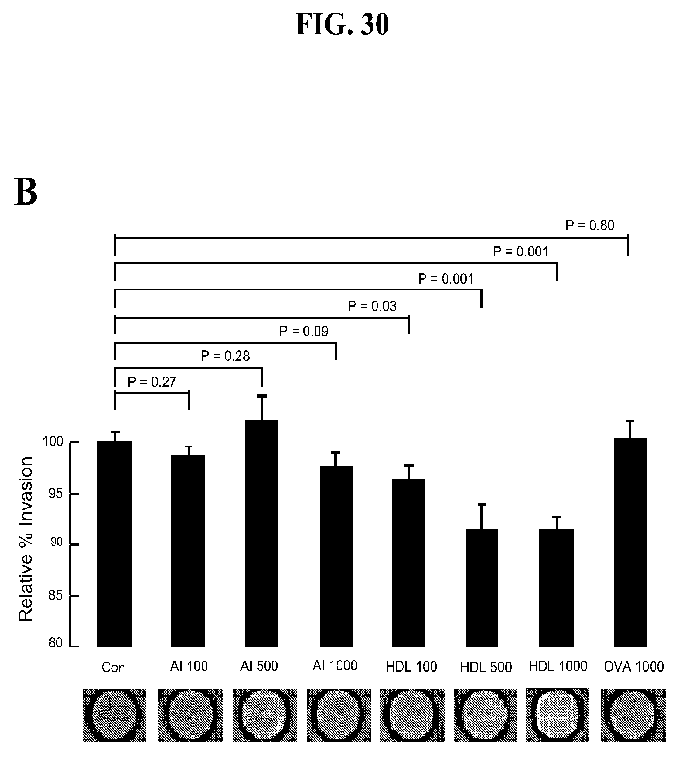

Figure 30 shows the effects of apoA1 and HDL on B16F10 Melanoma wound healing-migration and invasion through a basement membrane. B16F10 melanoma cells were plated in 6 well dishes containing a polymer strip which was removed to create a clear zone devoid of cells. The wells were washed with serum free DMEM 3x and serum-free DMEM containing the indicated concentration of apo AI or HDL. In addition, 4ug/ml of Mitomycin C (to block cellular proliferation) was added to each well so that only migration/wound healing would be observed. Cells were photographed every 15 minutes using a 5x objective and live imaging (37C, 5% CO2). Five fields per condition were examined. Images at time zero and time at 45 hours were used to calculate the area of wound closure. Initial total area devoid of cells for each condition was determined using the image analysis program Image J (NIH) as was the area devoid of cells in each condition at 45 h after wounding. Area of wound closure determined for the control served as 100% and the relative wound healing for each condition was determined by dividing the area of wound closure for each condition by the area of wound closure of the control cells. The results are shown in Figure 30A. Data presented represent the mean±SD of at least five photographic fields per condition. Significance was determined by two-tailed student t test. Representative photographs at time 0 and at 45 h after wounding are in the panel to the right and the experimental condition and concentration of apo AI or HDL is indicated. These experimental results are representative to two other identical experiments.

B16F10 melanoma cells 100,000 cells in 400ul of serum-free DMEM containing either apo AI or HDL at the indicated concentration/ml were placed in the upper chamber of a modified Boyden chamber. Total protein in each well except for the control (Con) was 400ug and was adjusted by the addition of ovalbumin to either the apo AI or HDL containing wells if needed. The bottom well of the Boyden chamber was filled with 0.8ml of serum-free DMEM. Cells were incubated for 30h after which the upper chambers were "scrubbed to remove all cells in the upper well so that only cells which had invaded the 1% basement membrane and moved to the other side of the filter would be observed. Filters were then fixed and stained with quick-fix. Filters were photographed at 5X and the total area covered by the stained cells was calculated by the imaging core at CCF using Image Pro software. The results are shown in Figure 30B. The area covered by cells on the control filter was designated as 100% invasion and cell areas were determined for all other experimental conditions and compared to that of the control cells. Representative photographs of stained filters for each condition are below the indicated condition in Figure 30B. Data presented represent the mean±SD of three filters per condition. Significance was determined by two-tailed student t test.

Collectively, the data in Figure 30 shows that HDL directly inhibits cancer cell migration and tissue invasion potential.

## Claims

1. A composition comprising a therapeutic agent and/or a nucleic acid sequence encoding said therapeutic agent, wherein said therapeutic agent comprises: i) apolipoprotein A-1 (ApoA1) or biologically active fragment thereof for use in a method of treating cancer in a subject through immunomodulatory effects on innate immune cells of said subject.

2. The composition for use according to Claim 1, wherein said therapeutic agent comprises a high-density lipoprotein (HDL) or HDL mimetic.

3. The composition for use according to Claim 1, wherein said therapeutic agent comprises preproapoliprotein (preproApoA1).

4. The composition for use according to Claim 1, wherein said composition is injected and/or infused into said subject.

5. The composition for use according to Claim 1, wherein said nucleic acid sequence comprises an ApoA1 mRNA sequence.

6. The composition for use according to Claim 1, wherein said nucleic acid sequence comprises a DNA sequence encoding said therapeutic agent.

7. The composition for use according to Claim 1, wherein said nucleic acid sequence comprises an expression vector.

8. The composition for use according to Claim 1, wherein said therapeutic agent further comprises ii) an induction agent that induces increased endogenous expression of apolipoprotein A-1 (ApoA1) in said subject, wherein said induction agent comprises one of the following:
- a nucleic acid sequence encoding the HNF-4 gene;
- a statin that is able to cause an increased expression of HDL or ABCA1, wherein said statin is Atorvastatin;
- niacin;
- a fibrate that is able to cause increased expression of HDL, wherein said fibrate is selected from the group consisting of: Fenofibrate, bezafibrate, gemfibrozil, and LY518674.

## Patentansprüche

1. Zusammensetzung, umfassend ein therapeutisches Mittel und/oder eine besagtes therapeutisches Mittel kodierende Nukleinsäuresequenz, wobei besagtes therapeutisches Mittel umfasst: i) Apolipoprotein A-1 (ApoAl) oder ein biologisch aktives Fragment davon zur Verwendung in einem Verfahren zur Behandlung von Krebs bei einem Subjekt durch immunmodulatorische Effekte auf angeborene Immunzellen des besagten Subjekts.

2. Zusammensetzung zur Verwendung nach Anspruch 1, wobei besagtes therapeutisches Mittel ein Lipoprotein erhöhter Dichte (HDL) oder HDL-Nachahmer umfasst.

3. Zusammensetzung zur Verwendung nach Anspruch 1, wobei besagtes therapeutisches Mittel Preproapoliprotein (preproApoA1) umfasst.

4. Zusammensetzung zur Verwendung nach Anspruch 1, wobei besagte Zusammensetzung in besagtes Subjekt injiziert und oder per Infusion verabreicht wird.

5. Zusammensetzung zur Verwendung nach Anspruch 1, wobei besagte Nukleinsäuresequenz eine ApoA1 mRNA-Sequenz umfasst.

6. Zusammensetzung zur Verwendung nach Anspruch 1, wobei besagte Nukleinsäuresequenz eine besagtes therapeutisches Mittel kodierende DNS-Sequenz umfasst.

7. Zusammensetzung zur Verwendung nach Anspruch 1, wobei besagte Nukleinsäuresequenz einen Expressionsvektor umfasst.

8. Zusammensetzung zur Verwendung nach Anspruch 1, wobei besagtes therapeutisches Mittel des Weiteren umfasst ii) ein Induktionsmittel, das in besagtem Subjekt eine erhöhte endogene Expression von Apolipoprotein A-1 (ApoAl) induziert, wobei besagtes Induktionsmittel eines der folgenden umfasst:
- eine das HNF-4 Gen kodierende Nukleinsäuresequenz;
- ein Statin, das in der Lage ist eine erhöhte Expression von HDL oder ABCA1 zu bewirken, wobei besagtes Statin Atorvastatin ist;
- Niacin;
- ein Fibrat, das in der Lage ist eine erhöhte Expression von HDL zu bewirken, wobei besagtes Fibrat ausgewählt ist aus der Gruppe bestehend aus: Fenofibrat, Bezafibrat, Gemfibrozil und LY518674.

## Revendications

1. Composition comprenant un agent thérapeutique et/ou une séquence d'acides nucléiques codant ledit agent thérapeutique, où ledit agent thérapeutique comprend : i) l'apolipoprotéine A-1 (ApoA1) ou un fragment biologiquement actif de celle-ci pour utilisation dans une méthode de traitement du cancer chez un sujet via des effets immunomodulateurs sur des cellules immunitaires innées dudit sujet.

2. Composition pour utilisation selon la Revendication 1, où ledit agent thérapeutique comprend une lipoprotéine haute densité (HDL) ou un mimétique de HDL.

3. Composition pour utilisation selon la Revendication 1, où ledit agent thérapeutique comprend de la préproapoliprotéine (préproApoA1).

4. Composition pour utilisation selon le Revendication 1, où ladite composition est injectée et/ou perfusée audit sujet.

5. Composition pour utilisation selon la Revendication 1, où ladite séquence d'acides nucléiques comprend une séquence d'ARNm d'ApoA1.

6. Composition pour utilisation selon la Revendication 1, où ladite séquence d'acides nucléiques comprend une séquence d'ADN codant ledit agent thérapeutique.

7. Composition pour utilisation selon la Revendication 1, où ladite séquence d'acides nucléiques comprend un vecteur d'expression.

8. Composition pour utilisation selon la Revendication 1, où ledit agent thérapeutique comprend en outre ii) un agent d'induction qui induit une expression endogène supérieure de l'apolipoprotéine A-1 (ApoA1) chez ledit sujet, où ledit agent d'induction comprend l'un des suivantes :
- une séquence d'acides nucléique codant le gène HNF-4 ;
- une statine capable de déclencher une augmentation de l'expression de HDL ou d'ABCA1, où ladite statine est l'Atorvastatine ;
- la niacine ;
- un fibrate capable de déclencher une augmentation de l'expression de HDL, où ledit fibrate est choisi dans le groupe constitué par les suivants : fénofibrate, bézafibrate, gemfibrozil et LY518674.


### SEQUENCE LISTING

<110> Hazen, Stanley L. Zamanian, Maryam DiDonato, Joseph
<120> Compositions and Methods for Treating Cancer
<130> CCF-31710/WO-1/ORD
<150> US 61/436,030
   <151> 2011-01-25
<150> US 61/355,134
   <151> 2010-06-15
<160> 69
<170> PatentIn version 3.5
<210> 1
   <211> 243
   <212> PRT
   <213> Homo sapiens
<220>
   <221> MISC_FEATURE
   <222> (8)..(8)
   <223> Xaa is a tryptophan residue or an oxidant resistant residue
<220>
   <221> MISC_FEATURE
   <222> (50)..(50)
   <223> Xaa is a tryptophan residue or an oxidant resistant residue
<220>
   <221> MISC_FEATURE
   <222> (72)..(72)
   <223> Xaa is a tryptophan residue or an oxidant resistant residue
<220>
   <221> MISC_FEATURE
   <222> (108)..(108)
   <223> Xaa is a tryptophan residue or an oxidant resistant residue
<400> 1
   Asp Glu Pro Pro Gln Ser Gln Xaa Asp Arg Val Lys Asp Leu Ala Thr
   1               5                   10                  15
   Val Tyr Val Asp Val Leu Lys Asp Ser Gly Arg Asp Tyr Val Ser Gln
               20                  25                  30
   Phe Glu Gly Ser Ala Leu Gly Lys Gln Leu Asn Leu Lys Leu Leu Asp
           35                  40                  45
   Asn Xaa Asp Ser Val Thr Ser Thr Phe Ser Lys Leu Arg Glu Gln Leu
       50                  55                  60
   Gly Pro Val Thr Gln Glu Phe Xaa Asp Asn Leu Glu Lys Glu Thr Glu
   65                  70                  75                  80
   Gly Leu Arg Gln Glu Met Ser Lys Asp Leu Glu Glu Val Lys Ala Lys
                   85                  90                  95
   Val Gln Pro Tyr Leu Asp Asp Phe Gln Lys Lys Xaa Gln Glu Glu Met
               100                 105                 110
   Glu Leu Tyr Arg Gln Lys Val Glu Pro Leu Arg Ala Glu Leu Gln Glu
           115                 120                 125
   Gly Ala Arg Gln Lys Leu His Glu Leu Gln Glu Lys Leu Ser Pro Leu
       130                 135                 140
   Gly Glu Glu Met Arg Asp Arg Ala Arg Ala His Val Asp Ala Leu Arg
   145                 150                 155                 160
   Thr His Leu Ala Pro Tyr Ser Asp Glu Leu Arg Gln Arg Leu Ala Ala
                   165                 170                 175
   Arg Leu Glu Ala Leu Lys Glu Asn Gly Gly Ala Arg Leu Ala Glu Tyr
               180                 185                 190
   His Ala Lys Ala Thr Glu His Leu Ser Thr Leu Ser Glu Lys Ala Lys
           195                 200                 205
   Pro Ala Leu Glu Asp Leu Arg Gln Gly Leu Leu Pro Val Leu Glu Ser
       210                 215                 220
   Phe Lys Val Ser Phe Leu Ser Ala Leu Glu Glu Tyr Thr Lys Lys Leu
   225                 230                 235                 240
   Asn Thr Gln
<210> 2
   <211> 267
   <212> PRT
   <213> Homo sapiens
<220>
   <221> MISC_FEATURE
   <222> (22)..(22)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<220>
   <221> MISC_FEATURE
   <222> (74)..(74)
   <223> Xaa is a tryptophan residue or an oxidant resistant residue
<220>
   <221> MISC_FEATURE
   <222> (96)..(96)
   <223> Xaa is a tryptophan residue or an oxidant resistant residue
<220>
   <221> MISC_FEATURE
   <222> (132)..(132)
   <223> Xaa is a tryptophan residue or an oxidant resistant residue
<400> 2
   Met Lys Ala Ala Val Leu Thr Leu Ala Val Leu Phe Leu Thr Gly Ser
   1               5                   10                  15
   Gln Ala Arg His Phe Xaa Gln Gln Asp Glu Pro Pro Gln Ser Pro Xaa
               20                  25                  30
   Asp Arg Val Lys Asp Leu Ala Thr Val Tyr Val Asp Val Leu Lys Asp
           35                  40                  45
   Ser Gly Arg Asp Tyr Val Ser Gln Phe Glu Gly Ser Ala Leu Gly Lys
       50                  55                  60
   Gln Leu Asn Leu Lys Leu Leu Asp Asn Xaa Asp Ser Val Thr Ser Thr
   65                  70                  75                  80
   Phe Ser Lys Leu Arg Glu Gln Leu Gly Pro Val Thr Gln Glu Phe Xaa
                   85                  90                  95
   Asp Asn Leu Glu Lys Glu Thr Glu Gly Leu Arg Gln Glu Met Ser Lys
               100                 105                 110
   Asp Leu Glu Glu Val Lys Ala Lys Val Gln Pro Tyr Leu Asp Asp Phe
           115                 120                 125
   Gln Lys Lys Xaa Gln Glu Glu Met Glu Leu Tyr Arg Gln Lys Val Glu
       130                 135                 140
   Pro Leu Arg Ala Glu Leu Gln Glu Gly Ala Arg Gln Lys Leu His Glu
   145                 150                 155                 160
   Leu Gln Glu Lys Leu Ser Pro Leu Gly Glu Glu Met Arg Asp Arg Ala
                   165                 170                 175
   Arg Ala His Val Asp Ala Leu Cys Thr His Leu Ala Pro Tyr Ser Asp
               180                 185                 190
   Glu Leu Arg Gln Arg Leu Ala Ala Arg Leu Glu Ala Leu Lys Glu Asn
           195                 200                 205
   Gly Gly Ala Arg Leu Ala Glu Tyr His Ala Lys Ala Thr Glu His Leu
       210                 215                 220
   Ser Thr Leu Ser Glu Lys Ala Lys Pro Ala Leu Glu Asp Leu Arg Gln
   225                 230                 235                 240
   Gly Leu Leu Pro Val Leu Glu Ser Phe Lys Val Ser Phe Leu Ser Ala
                   245                 250                 255
   Leu Glu Glu Tyr Thr Lys Lys Leu Asn Thr Gln
               260                 265
<210> 3
   <211> 267
   <212> PRT
   <213> Homo sapiens
<220>
   <221> MISC_FEATURE
   <222> (22)..(22)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<220>
   <221> MISC_FEATURE
   <222> (74)..(74)
   <223> Xaa is a tryptophan residue or an oxidant resistant residue
<220>
   <221> MISC_FEATURE
   <222> (96)..(96)
   <223> Xaa is a tryptophan residue or an oxidant resistant residue
<220>
   <221> MISC_FEATURE
   <222> (132)..(132)
   <223> Xaa is a tryptophan residue or an oxidant resistant residue
<400> 3
   Met Lys Ala Ala Val Leu Thr Leu Ala Val Leu Phe Leu Thr Gly Ser
   1               5                   10                  15
   Gln Ala Arg His Phe Xaa Gln Gln Asp Glu Pro Pro Gln Ser Pro Xaa
               20                  25                  30
   Asp Arg Val Lys Asp Leu Ala Thr Val Tyr Val Asp Val Leu Lys Asp
           35                  40                  45
   Ser Gly Arg Asp Tyr Val Ser Gln Phe Glu Gly Ser Ala Leu Gly Lys
       50                  55                  60
   Gln Leu Asn Leu Lys Leu Leu Asp Asn Xaa Asp Ser Val Thr Ser Thr
   65                  70                  75                  80
   Phe Ser Lys Leu Arg Glu Gln Leu Gly Pro Val Thr Gln Glu Phe Xaa
                   85                  90                  95
   Asp Asn Leu Glu Lys Glu Thr Glu Gly Leu Arg Gln Glu Met Ser Lys
               100                 105                 110
   Asp Leu Glu Glu Val Lys Ala Lys Val Gln Pro Tyr Leu Asp Asp Phe
           115                 120                 125
   Gln Lys Lys Xaa Gln Glu Glu Met Glu Leu Tyr Arg Gln Lys Val Glu
       130                 135                 140
   Pro Leu Arg Ala Glu Leu Gln Glu Gly Ala Arg Gln Lys Leu His Glu
   145                 150                 155                 160
   Leu Gln Glu Lys Leu Cys Pro Leu Gly Glu Glu Met Arg Asp Arg Ala
                   165                 170                 175
   Arg Ala His Val Asp Ala Leu Cys Thr His Leu Ala Pro Tyr Ser Asp
               180                 185                 190
   Glu Leu Arg Gln Arg Leu Ala Ala Arg Leu Glu Ala Leu Lys Glu Asn
           195                 200                 205
   Gly Gly Ala Arg Leu Ala Glu Tyr His Ala Lys Ala Thr Glu His Leu
       210                 215                 220
   Ser Thr Leu Ser Glu Lys Ala Lys Pro Ala Leu Glu Asp Leu Arg Gln
   225                 230                 235                 240
   Gly Leu Leu Pro Val Leu Glu Ser Phe Lys Val Ser Phe Leu Ser Ala
                   245                 250                 255
   Leu Glu Glu Tyr Thr Lys Lys Leu Asn Thr Gln
               260                 265
<210> 4
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 4
   Asp Xaa Leu Lys Ala Phe Tyr Asp Lys Val Ala Glu Lys Leu Lys Glu
   1               5                   10                  15
   Ala Phe
<210> 5
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 5
   Asp Xaa Phe Lys Ala Phe Tyr Asp Lys Val Ala Glu Lys Leu Lys Glu
   1               5                   10                  15
   Ala Phe
<210> 6
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 6
   Asp Xaa Leu Lys Ala Phe Tyr Asp Lys Val Ala Glu Lys Phe Lys Glu
   1               5                   10                  15
   Ala Phe
<210> 7
   <211> 18
   <212> PRT
   <213> Artificial Sequence
   <220>
   <223> Synthetic
<220>
   <221> MISC_FEATURE
   <222> (2)..(2)
   <223> Xaa is a tryptophan residue or an oxidant resistant residue
<400> 7
   Asp Xaa Phe Lys Ala Phe Tyr Asp Lys Val Ala Glu Lys Phe Lys Glu
   1               5                   10                  15
   Ala Phe
<210> 8
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 8
   Asp Xaa Leu Lys Ala Phe Tyr Asp Lys Val Phe Glu Lys Phe Lys Glu
   1               5                   10                  15
   Phe Phe
<210> 9
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 9
   Asp Xaa Leu Lys Ala Phe Tyr Asp Lys Phe Phe Glu Lys Phe Lys Glu
   1               5                   10                  15
   Phe Phe
<210> 10
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 10
   Asp Xaa Phe Lys Ala Phe Tyr Asp Lys Phe Phe Glu Lys Phe Lys Glu
   1               5                   10                  15
   Phe Phe
<210> 11
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 11
   Asp Xaa Leu Lys Ala Phe Tyr Asp Lys Val Phe Glu Lys Phe Lys Glu
   1               5                   10                  15
   Ala Phe
<210> 12
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 12
   Asp Xaa Leu Lys Ala Phe Tyr Asp Lys Val Phe Glu Lys Phe Lys Glu
   1 5 10 15
   Ala Phe
<210> 13
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 13
   Asp Xaa Leu Lys Ala Phe Tyr Asp Lys Val Phe Glu Lys Leu Lys Glu
   1 5 10 15
   Phe Phe
<210> 14
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 14
   Asp Xaa Leu Lys Ala Phe Tyr Asp Lys Val Ala Glu Lys Phe Lys Glu
   1 5 10 15
   Phe Phe
<210> 15
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 15
   Asp Xaa Leu Lys Ala Phe Tyr Asp Lys Val Phe Glu Lys Phe Lys Glu
   1               5                   10                  15
   Phe Phe
<210> 16
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 16
   Glu Xaa Leu Lys Leu Phe Tyr Glu Lys Val Leu Glu Lys Phe Lys Glu
   1               5                   10                  15
   Ala Phe
<210> 17
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 17
   Glu Xaa Leu Lys Ala Phe Tyr Asp Lys Val Ala Glu Lys Phe Lys Glu
   1               5                   10                  15
   Ala Phe
<210> 18
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 18
   Glu Xaa Leu Lys Ala Phe Tyr Asp Lys Val Ala Glu Lys Leu Lys Glu
   1               5                   10                  15
   Phe Phe
<210> 19
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 19
   Glu Xaa Leu Lys Ala Phe Tyr Asp Lys Val Phe Glu Lys Phe Lys Glu
   1               5                   10                  15
   Ala Phe
<210> 20
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 20
   Glu Xaa Leu Lys Ala Phe Tyr Asp Lys Val Phe Glu Lys Leu Lys Glu
   1 5 10 15
   Phe Phe
<210> 21
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 21
   Glu Xaa Leu Lys Ala Phe Tyr Asp Lys Val Ala Glu Lys Phe Lys Glu
   1 5 10 15
   Phe Phe
<210> 22
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 22
   Glu Xaa Leu Lys Ala Phe Tyr Asp Lys Val Phe Glu Lys Phe Lys Glu
   1 5 10 15
   Phe Phe
<210> 23
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 23
   Asp Xaa Leu Lys Ala Leu Tyr Asp Lys Val Ala Glu Lys Leu Lys Glu
   1 5 10 15
   Ala Leu
<210> 24
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 24
   Asp Xaa Phe Lys Ala Phe Tyr Glu Lys Val Ala Glu Lys Leu Lys Glu
   1 5 10 15
   Phe Phe
<210> 25
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 25
   Asp Xaa Phe Lys Ala Phe Tyr Glu Lys Phe Phe Glu Lys Phe Lys Glu
   1 5 10 15
   Phe Phe
<210> 26
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 26
   Glu Xaa Leu Lys Ala Leu Tyr Glu Lys Val Ala Glu Lys Leu Lys Glu
   1               5                   10                  15
   Ala Leu
<210> 27
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 27
   Glu Xaa Leu Lys Ala Phe Tyr Glu Lys Val Ala Glu Lys Leu Lys Glu
   1               5                   10                  15
   Ala Phe
<210> 28
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 28
   Glu Xaa Phe Lys Ala Phe Tyr Glu Lys Val Ala Glu Lys Leu Lys Glu
   1               5                   10                  15
   Phe Phe
<210> 29
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 29
   Glu Xaa Leu Lys Ala Phe Tyr Glu Lys Val Phe Glu Lys Phe Lys Glu
   1               5                   10                  15
   Phe Phe
<210> 30
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 30
   Glu Xaa Leu Lys Ala Phe Tyr Glu Lys Phe Phe Glu Lys Phe Lys Glu
   1               5                   10                  15
   Phe Phe
<210> 31
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 31
   Glu Xaa Phe Lys Ala Phe Tyr Glu Lys Phe Phe Glu Lys Phe Lys Glu
   1 5 10 15
   Phe Phe
<210> 32
   <211> 18
   <212> PRT
   <213> Artificial Sequence
<220>
   <223> Synthetic
<220>
   <221> MISC_FEATURE
   <222> (6)..(6)
   <223> Xaa is a tryptophan residue or an oxidant resistant residue
<220>
   <221> MISC_FEATURE
   <222> (18)..(18)
   <223> Xaa is a tryptophan residue or an oxidant resistant residue
<400> 32
   Asp Phe Leu Lys Ala Xaa Tyr Asp Lys Val Ala Glu Lys Leu Lys Glu
   1 5 10 15
   Ala Xaa
<210> 33
   <211> 18
   <212> PRT
   <213> Artificial Sequence
<220>
   <223> Synthetic
<220>
   <221> MISC_FEATURE
   <222> (6)..(6)
   <223> Xaa is a tryptophan residue or an oxidant resistant residue
<220>
   <221> MISC_FEATURE
   <222> (18)..(18)
   <223> Xaa is a tryptophan residue or an oxidant resistant residue
<400> 33
   Glu Phe Leu Lys Ala Xaa Tyr Glu Lys Val Ala Glu Lys Leu Lys Glu
   1 5 10 15
   Ala Xaa
<210> 34
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (6)..(6)
   <223> Xaa is a tryptophan residue or an oxidant resistant residue
<220>
   <221> MISC_FEATURE
   <222> (17)..(18)
   <223> Xaa is a tryptophan residue or an oxidant resistant residue
<400> 34
   Asp Phe Xaa Lys Ala Xaa Tyr Asp Lys Val Ala Glu Lys Leu Lys Glu
   1               5                   10                  15
   Xaa Xaa
<210> 35
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (6)..(6)
   <223> Xaa is a tryptophan residue or an oxidant resistant residue
<220>
   <221> MISC_FEATURE
   <222> (17)..(18)
   <223> Xaa is a tryptophan residue or an oxidant resistant residue
<400> 35
   Glu Phe Xaa Lys Ala Xaa Tyr Glu Lys Val Ala Glu Lys Leu Lys Glu
   1 5 10 15
   Xaa Xaa
<210> 36
   <211> 18
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<400> 36
   Asp Lys Leu Lys Ala Phe Tyr Asp Lys Val Phe Glu Xaa Ala Lys Glu
   1 5 10 15
   Ala Phe
<210> 37
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 37
   Asp Lys Xaa Lys Ala Val Tyr Asp Lys Phe Ala Glu Ala Phe Lys Glu
   1 5 10 15
   Phe Leu
<210> 38
   <211> 18
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<400> 38
   Glu Lys Leu Lys Ala Phe Tyr Glu Lys Val Phe Glu Xaa Ala Lys Glu
   1               5                   10                  15
   Ala Phe
<210> 39
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 39
   Glu Lys Xaa Lys Ala Val Tyr Glu Lys Phe Ala Glu Ala Phe Lys Glu
   1               5                   10                  15
   Phe Leu
<210> 40
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 40
   Asp Xaa Leu Lys Ala Phe Val Asp Lys Phe Ala Glu Lys Phe Lys Glu
   1               5                   10                  15
   Ala Tyr
<210> 41
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 41
   Glu Lys Xaa Lys Ala Val Tyr Glu Lys Phe Ala Glu Ala Phe Lys Glu
   1               5                   10                  15
   Phe Leu
<210> 42
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 42
   Asp Xaa Leu Lys Ala Phe Val Tyr Asp Lys Val Phe Lys Leu Lys Glu
   1               5                   10                  15
   Phe Phe
<210> 43
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 43
   Glu Xaa Leu Lys Ala Phe Val Tyr Glu Lys Val Phe Lys Leu Lys Glu
   1               5                   10                  15
   Phe Phe
<210> 44
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 44
   Asp Xaa Leu Arg Ala Phe Tyr Asp Lys Val Ala Glu Lys Leu Lys Glu
   1               5                   10                  15
   Ala Phe
<210> 45
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 45
   Glu Xaa Leu Arg Ala Phe Tyr Glu Lys Val Ala Glu Lys Leu Lys Glu
   1               5                   10                  15
   Ala Phe
<210> 46
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 46
   Asp Xaa Leu Lys Ala Phe Tyr Asp Arg Val Ala Glu Lys Leu Lys Glu
   1 5 10 15
   Ala Phe
<210> 47
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 47
   Glu Xaa Leu Lys Ala Phe Tyr Glu Arg Val Ala Glu Lys Leu Lys Glu
   1 5 10 15
   Ala Phe
<210> 48
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 48
   Asp Xaa Leu Lys Ala Phe Tyr Asp Lys Val Ala Glu Arg Leu Lys Glu
   1 5 10 15
   Ala Phe
<210> 49
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 49
   Glu Xaa Leu Lys Ala Phe Tyr Glu Lys Val Ala Glu Arg Leu Lys Glu
   1               5                   10                  15
   Ala Phe
<210> 50
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 50
   Asp Xaa Leu Lys Ala Phe Tyr Asp Lys Val Ala Glu Lys Leu Arg Glu
   1               5                   10                  15
   Ala Phe
<210> 51
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 51
   Glu Xaa Leu Lys Ala Phe Tyr Glu Lys Val Ala Glu Lys Leu Arg Glu
   1               5                   10                  15
   Ala Phe
<210> 52
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 52
   Asp Xaa Leu Lys Ala Phe Tyr Asp Arg Val Ala Glu Arg Leu Lys Glu
   1               5                   10                  15
   Ala Phe
<210> 53
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 53
   Glu Xaa Leu Lys Ala Phe Tyr Glu Arg Val Ala Glu Arg Leu Lys Glu
   1               5                   10                  15
   Ala Phe
<210> 54
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 54
   Asp Xaa Leu Arg Ala Phe Tyr Asp Lys Val Ala Glu Lys Leu Arg Glu
   1               5                   10                  15
   Ala Phe
<210> 55
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 55
   Glu Xaa Leu Arg Ala Phe Tyr Glu Lys Val Ala Glu Lys Leu Arg Glu
   1               5                   10                  15
   Ala Phe
<210> 56
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 56
   Asp Xaa Leu Arg Ala Phe Tyr Asp Arg Val Ala Glu Lys Leu Lys Glu
   1               5                   10                  15
   Ala Phe
<210> 57
   <211> 17
   <212> PRT
   <213> Artificial Sequence
<220>
   <223> Synthetic
<220>
   <221> MISC_FEATURE
   <222> (1)..(1)
   <223> Xaa is a tryptophan residue or an oxidant resistant residue
<400> 57
   Xaa Leu Arg Ala Phe Tyr Glu Arg Val Ala Glu Lys Leu Lys Glu Ala
   1 5 10 15
   Phe
<210> 58
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 58
   Asp Xaa Leu Lys Ala Phe Tyr Asp Lys Val Ala Glu Arg Leu Arg Glu
   1 5 10 15
   Ala Phe
<210> 59
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 59
   Glu Xaa Leu Lys Ala Phe Tyr Glu Lys Val Ala Glu Arg Leu Arg Glu
   1 5 10 15
   Ala Phe
<210> 60
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 60
   Asp Xaa Leu Arg Ala Phe Tyr Asp Lys Val Ala Glu Arg Leu Lys Glu
   1               5                   10                  15
   Ala Phe
<210> 61
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 61
   Glu Xaa Leu Arg Ala Phe Tyr Glu Lys Val Ala Glu Arg Leu Lys Glu
   1               5                   10                  15
   Ala Phe
<210> 62
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 62
   Asp Xaa Leu Lys Ala Phe Tyr Asp Lys Val Ala Glu Lys Leu Lys Glu
   1               5                   10                  15
   Ala Phe Pro Asp Xaa Leu Lys Ala Phe Tyr Asp Lys Val Ala Glu Lys
               20                  25                  30
   Leu Lys Glu Ala Phe
           35
<210> 63
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 63
   Asp Xaa Leu Lys Ala Phe Tyr Asp Lys Val Ala Glu Lys Leu Lys Glu
   1               5                   10                  15
   Phe Phe Pro Asp Xaa Leu Lys Ala Phe Tyr Asp Lys Val Ala Glu Lys
               20                  25                  30
   Leu Lys Glu Phe Phe
           35
<210> 64
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 64
   Asp Xaa Phe Lys Ala Phe Tyr Asp Lys Val Ala Glu Lys Leu Lys Glu
   1               5                   10                  15
   Ala Phe Pro Asp Xaa Phe Lys Ala Phe Tyr Asp Lys Val Ala Glu Lys
               20                  25                  30
   Leu Lys Glu Ala Phe
           35
<210> 65
   <211> 37
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<400> 65
   Asp Lys Leu Lys Ala Phe Tyr Asp Lys Val Phe Glu Xaa Ala Lys Glu
   1               5                   10                  15
   Ala Phe Pro Asp Lys Leu Lys Ala Phe Tyr Asp Lys Val Phe Glu Xaa
               20                  25                  30
   Leu Lys Glu Ala Phe
           35
<210> 66
   <211> 37
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (22)..(22)
   <223> Xaa is a tryptophan residue or an oxidant resistant residue
<400> 66
   Asp Lys Xaa Lys Ala Val Tyr Asp Lys Phe Ala Glu Ala Phe Lys Glu
   1               5                   10                  15
   Phe Leu Pro Asp Lys Xaa Lys Ala Val Tyr Asp Lys Phe Ala Glu Ala
               20                  25                  30
   Phe Lys Glu Phe Leu
           35
<210> 67
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 67
   Asp Xaa Phe Lys Ala Phe Tyr Asp Lys Val Ala Glu Lys Phe Lys Glu
   1               5                   10                  15
   Ala Phe Pro Asp Xaa Phe Lys Ala Phe Tyr Asp Lys Val Ala Glu Lys
               20                  25                  30
   Phe Lys Glu Ala Phe
           35
<210> 68
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 68
   Asp Xaa Leu Lys Ala Phe Val Tyr Asp Lys Val Phe Lys Leu Lys Glu
   1               5                   10                  15
   Phe Phe Pro Asp Xaa Leu Lys Ala Phe Val Tyr Asp Lys Val Phe Lys
               20                  25                  30
   Leu Lys Glu Phe Phe
           35
<210> 69
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 69
   Asp Xaa Leu Lys Ala Phe Tyr Asp Lys Phe Ala Glu Lys Phe Lys Glu
   1               5                   10                  15
   Phe Phe Pro Asp Xaa Leu Lys Ala Phe Tyr Asp Lys Phe Ala Glu Lys
               20                  25                  30
   Phe Lys Glu Phe Phe
           35

### SEQUENCE LISTING

<110> Hazen, Stanley L.
   Zamanian, Maryam
   DiDonato, Joseph
<120> Compositions and Methods for Treating Cancer
<130> CCF-31710/WO-1/ORD
<150> US 61/436,030
   <151> 2011-01-25
<150> US 61/355,134
   <151> 2010-06-15
<160> 69
<170> PatentIn version 3.5
<210> 1
   <211> 243
   <212> PRT
   <213> Homo sapiens
<220>
   <221> MISC_FEATURE
   <222> (8)..(8)
   <223> Xaa is a tryptophan residue or an oxidant resistant residue
<220>
   <221> MISC_FEATURE
   <222> (50)..(50)
   <223> Xaa is a tryptophan residue or an oxidant resistant residue
<220>
   <221> MISC_FEATURE
   <222> (72)..(72)
   <223> Xaa is a tryptophan residue or an oxidant resistant residue
<220>
   <221> MISC_FEATURE
   <222> (108)..(108)
   <223> Xaa is a tryptophan residue or an oxidant resistant residue
<400> 1
   Asp Glu Pro Pro Gln Ser Gln Xaa Asp Arg Val Lys Asp Leu Ala Thr
   1               5                   10                  15
   Val Tyr Val Asp Val Leu Lys Asp Ser Gly Arg Asp Tyr Val Ser Gln
               20                  25                  30
   Phe Glu Gly Ser Ala Leu Gly Lys Gln Leu Asn Leu Lys Leu Leu Asp
           35                  40                  45
   Asn Xaa Asp Ser Val Thr Ser Thr Phe Ser Lys Leu Arg Glu Gln Leu
       50                  55                  60
   Gly Pro Val Thr Gln Glu Phe Xaa Asp Asn Leu Glu Lys Glu Thr Glu
   65                  70                  75                  80
   Gly Leu Arg Gln Glu Met Ser Lys Asp Leu Glu Glu Val Lys Ala Lys
                   85                  90                  95
   Val Gln Pro Tyr Leu Asp Asp Phe Gln Lys Lys Xaa Gln Glu Glu Met
               100                 105                 110
   Glu Leu Tyr Arg Gln Lys Val Glu Pro Leu Arg Ala Glu Leu Gln Glu
           115                 120                 125
   Gly Ala Arg Gln Lys Leu His Glu Leu Gln Glu Lys Leu Ser Pro Leu
       130                 135                 140
   Gly Glu Glu Met Arg Asp Arg Ala Arg Ala His Val Asp Ala Leu Arg
   145                 150                 155                 160
   Thr His Leu Ala Pro Tyr Ser Asp Glu Leu Arg Gln Arg Leu Ala Ala
                   165                 170                 175
   Arg Leu Glu Ala Leu Lys Glu Asn Gly Gly Ala Arg Leu Ala Glu Tyr
               180                 185                 190
   His Ala Lys Ala Thr Glu His Leu Ser Thr Leu Ser Glu Lys Ala Lys
           195                 200                 205
   Pro Ala Leu Glu Asp Leu Arg Gln Gly Leu Leu Pro Val Leu Glu Ser
       210                 215                 220
   Phe Lys Val Ser Phe Leu Ser Ala Leu Glu Glu Tyr Thr Lys Lys Leu
   225                 230                 235                 240
   Asn Thr Gln
<210> 2
   <211> 267
   <212> PRT
   <213> Homo sapiens
<220>
   <221> MISC_FEATURE
   <222> (22)..(22)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<220>
   <221> MISC_FEATURE
   <222> (74)..(74)
   <223> Xaa is a tryptophan residue or an oxidant resistant residue
<220>
   <221> MISC_FEATURE
   <222> (96)..(96)
   <223> Xaa is a tryptophan residue or an oxidant resistant residue
<220>
   <221> MISC_FEATURE
   <222> (132)..(132)
   <223> Xaa is a tryptophan residue or an oxidant resistant residue
<400> 2
   Met Lys Ala Ala Val Leu Thr Leu Ala Val Leu Phe Leu Thr Gly Ser
   1               5                   10                  15
   Gln Ala Arg His Phe Xaa Gln Gln Asp Glu Pro Pro Gln Ser Pro Xaa
               20                  25                  30
   Asp Arg Val Lys Asp Leu Ala Thr Val Tyr Val Asp Val Leu Lys Asp
           35                  40                  45
   Ser Gly Arg Asp Tyr Val Ser Gln Phe Glu Gly Ser Ala Leu Gly Lys
       50                  55                  60
   Gln Leu Asn Leu Lys Leu Leu Asp Asn Xaa Asp Ser Val Thr Ser Thr
   65                  70                  75                  80
   Phe Ser Lys Leu Arg Glu Gln Leu Gly Pro Val Thr Gln Glu Phe Xaa
                   85                  90                  95
   Asp Asn Leu Glu Lys Glu Thr Glu Gly Leu Arg Gln Glu Met Ser Lys
               100                 105                 110
   Asp Leu Glu Glu Val Lys Ala Lys Val Gln Pro Tyr Leu Asp Asp Phe
           115                 120                 125
   Gln Lys Lys Xaa Gln Glu Glu Met Glu Leu Tyr Arg Gln Lys Val Glu
       130                 135                 140
   Pro Leu Arg Ala Glu Leu Gln Glu Gly Ala Arg Gln Lys Leu His Glu
   145                 150                 155                 160
   Leu Gln Glu Lys Leu Ser Pro Leu Gly Glu Glu Met Arg Asp Arg Ala
                   165                 170                 175
   Arg Ala His Val Asp Ala Leu Cys Thr His Leu Ala Pro Tyr Ser Asp
               180                 185                 190
   Glu Leu Arg Gln Arg Leu Ala Ala Arg Leu Glu Ala Leu Lys Glu Asn
           195                 200                 205
   Gly Gly Ala Arg Leu Ala Glu Tyr His Ala Lys Ala Thr Glu His Leu
       210                 215                 220
   Ser Thr Leu Ser Glu Lys Ala Lys Pro Ala Leu Glu Asp Leu Arg Gln
   225                 230                 235                 240
   Gly Leu Leu Pro Val Leu Glu Ser Phe Lys Val Ser Phe Leu Ser Ala
                   245                 250                 255
   Leu Glu Glu Tyr Thr Lys Lys Leu Asn Thr Gln
               260                 265
<210> 3
   <211> 267
   <212> PRT
   <213> Homo sapiens
<220>
   <221> MISC_FEATURE
   <222> (22)..(22)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<220>
   <221> MISC_FEATURE
   <222> (74)..(74)
   <223> Xaa is a tryptophan residue or an oxidant resistant residue
<220>
   <221> MISC_FEATURE
   <222> (96)..(96)
   <223> Xaa is a tryptophan residue or an oxidant resistant residue
<220>
   <221> MISC_FEATURE
   <222> (132)..(132)
   <223> Xaa is a tryptophan residue or an oxidant resistant residue
<400> 3
   Met Lys Ala Ala Val Leu Thr Leu Ala Val Leu Phe Leu Thr Gly Ser
   1               5                   10                  15
   Gln Ala Arg His Phe Xaa Gln Gln Asp Glu Pro Pro Gln Ser Pro Xaa
               20                  25                  30
   Asp Arg Val Lys Asp Leu Ala Thr Val Tyr Val Asp Val Leu Lys Asp
           35                  40                  45
   Ser Gly Arg Asp Tyr Val Ser Gln Phe Glu Gly Ser Ala Leu Gly Lys
       50                  55                  60
   Gln Leu Asn Leu Lys Leu Leu Asp Asn Xaa Asp Ser Val Thr Ser Thr
   65                  70                  75                  80
   Phe Ser Lys Leu Arg Glu Gln Leu Gly Pro Val Thr Gln Glu Phe Xaa
                   85                  90                  95
   Asp Asn Leu Glu Lys Glu Thr Glu Gly Leu Arg Gln Glu Met Ser Lys
               100                 105                 110
   Asp Leu Glu Glu Val Lys Ala Lys Val Gln Pro Tyr Leu Asp Asp Phe
           115                 120                 125
   Gln Lys Lys Xaa Gln Glu Glu Met Glu Leu Tyr Arg Gln Lys Val Glu
       130                 135                 140
   Pro Leu Arg Ala Glu Leu Gln Glu Gly Ala Arg Gln Lys Leu His Glu
   145                 150                 155                 160
   Leu Gln Glu Lys Leu Cys Pro Leu Gly Glu Glu Met Arg Asp Arg Ala
                   165                 170                 175
   Arg Ala His Val Asp Ala Leu Cys Thr His Leu Ala Pro Tyr Ser Asp
               180                 185                 190
   Glu Leu Arg Gln Arg Leu Ala Ala Arg Leu Glu Ala Leu Lys Glu Asn
           195                 200                 205
   Gly Gly Ala Arg Leu Ala Glu Tyr His Ala Lys Ala Thr Glu His Leu
       210                 215                 220
   Ser Thr Leu Ser Glu Lys Ala Lys Pro Ala Leu Glu Asp Leu Arg Gln
   225                 230                 235                 240
   Gly Leu Leu Pro Val Leu Glu Ser Phe Lys Val Ser Phe Leu Ser Ala
                   245                 250                 255
   Leu Glu Glu Tyr Thr Lys Lys Leu Asn Thr Gln
               260                 265
<210> 4
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 4
   Asp Xaa Leu Lys Ala Phe Tyr Asp Lys Val Ala Glu Lys Leu Lys Glu
   1               5                   10                  15
   Ala Phe
<210> 5
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 5
   Asp Xaa Phe Lys Ala Phe Tyr Asp Lys Val Ala Glu Lys Leu Lys Glu
   1               5                   10                  15
   Ala Phe
<210> 6
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 6
   Asp Xaa Leu Lys Ala Phe Tyr Asp Lys Val Ala Glu Lys Phe Lys Glu
   1               5                   10                  15
   Ala Phe
<210> 7
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 7
   Asp Xaa Phe Lys Ala Phe Tyr Asp Lys Val Ala Glu Lys Phe Lys Glu
   1               5                   10                  15
   Ala Phe
<210> 8
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 8
   Asp Xaa Leu Lys Ala Phe Tyr Asp Lys Val Phe Glu Lys Phe Lys Glu
   1               5                   10                  15
   Phe Phe
<210> 9
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 9
   Asp Xaa Leu Lys Ala Phe Tyr Asp Lys Phe Phe Glu Lys Phe Lys Glu
   1               5                   10                  15
   Phe Phe
<210> 10
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 10
   Asp Xaa Phe Lys Ala Phe Tyr Asp Lys Phe Phe Glu Lys Phe Lys Glu
   1               5                   10                  15
   Phe Phe
<210> 11
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 11
   Asp Xaa Leu Lys Ala Phe Tyr Asp Lys Val Phe Glu Lys Phe Lys Glu
   1               5                   10                  15
   Ala Phe
<210> 12
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 12
   Asp Xaa Leu Lys Ala Phe Tyr Asp Lys Val Phe Glu Lys Phe Lys Glu
   1 5 10 15
   Ala Phe
<210> 13
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 13
   Asp Xaa Leu Lys Ala Phe Tyr Asp Lys Val Phe Glu Lys Leu Lys Glu
   1 5 10 15
   Phe Phe
<210> 14
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 14
   Asp Xaa Leu Lys Ala Phe Tyr Asp Lys Val Ala Glu Lys Phe Lys Glu
   1 5 10 15
   Phe Phe
<210> 15
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 15
   Asp Xaa Leu Lys Ala Phe Tyr Asp Lys Val Phe Glu Lys Phe Lys Glu
   1               5                   10                  15
   Phe Phe
<210> 16
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 16
   Glu Xaa Leu Lys Leu Phe Tyr Glu Lys Val Leu Glu Lys Phe Lys Glu
   1               5                   10                  15
   Ala Phe
<210> 17
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 17
   Glu Xaa Leu Lys Ala Phe Tyr Asp Lys Val Ala Glu Lys Phe Lys Glu
   1               5                   10                  15
   Ala Phe
<210> 18
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 18
   Glu Xaa Leu Lys Ala Phe Tyr Asp Lys Val Ala Glu Lys Leu Lys Glu
   1               5                   10                  15
   Phe Phe
<210> 19
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 19
   Glu Xaa Leu Lys Ala Phe Tyr Asp Lys Val Phe Glu Lys Phe Lys Glu
   1               5                   10                  15
   Ala Phe
<210> 20
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 20
   Glu Xaa Leu Lys Ala Phe Tyr Asp Lys Val Phe Glu Lys Leu Lys Glu
   1 5 10 15
   Phe Phe
<210> 21
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 21
   Glu Xaa Leu Lys Ala Phe Tyr Asp Lys Val Ala Glu Lys Phe Lys Glu
   1 5 10 15
   Phe Phe
<210> 22
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 22
   Glu Xaa Leu Lys Ala Phe Tyr Asp Lys Val Phe Glu Lys Phe Lys Glu
   1 5 10 15
   Phe Phe
<210> 23
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 23
   Asp Xaa Leu Lys Ala Leu Tyr Asp Lys Val Ala Glu Lys Leu Lys Glu
   1               5                   10                  15
   Ala Leu
<210> 24
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 24
   Asp Xaa Phe Lys Ala Phe Tyr Glu Lys Val Ala Glu Lys Leu Lys Glu
   1               5                   10                  15
   Phe Phe
<210> 25
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 25
   Asp Xaa Phe Lys Ala Phe Tyr Glu Lys Phe Phe Glu Lys Phe Lys Glu
   1               5                   10                  15
   Phe Phe
<210> 26
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 26
   Glu Xaa Leu Lys Ala Leu Tyr Glu Lys Val Ala Glu Lys Leu Lys Glu
   1               5                   10                  15
   Ala Leu
<210> 27
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 27
   Glu Xaa Leu Lys Ala Phe Tyr Glu Lys Val Ala Glu Lys Leu Lys Glu
   1               5                   10                  15
   Ala Phe
<210> 28
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 28
   Glu Xaa Phe Lys Ala Phe Tyr Glu Lys Val Ala Glu Lys Leu Lys Glu
   1               5                   10                  15
   Phe Phe
<210> 29
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 29
   Glu Xaa Leu Lys Ala Phe Tyr Glu Lys Val Phe Glu Lys Phe Lys Glu
   1               5                   10                  15
   Phe Phe
<210> 30
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 30
   Glu Xaa Leu Lys Ala Phe Tyr Glu Lys Phe Phe Glu Lys Phe Lys Glu
   1               5                   10                  15
   Phe Phe
<210> 31
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 31
   Glu Xaa Phe Lys Ala Phe Tyr Glu Lys Phe Phe Glu Lys Phe Lys Glu
   1 5 10 15
   Phe Phe
<210> 32
   <211> 18
   <212> PRT
   <213> Artificial Sequence
<220>
   <223> Synthetic
<220>
   <221> MISC_FEATURE
   <222> (6)..(6)
   <223> Xaa is a tryptophan residue or an oxidant resistant residue
<220>
   <221> MISC_FEATURE
   <222> (18)..(18)
   <223> Xaa is a tryptophan residue or an oxidant resistant residue
<400> 32
   Asp Phe Leu Lys Ala Xaa Tyr Asp Lys Val Ala Glu Lys Leu Lys Glu
   1 5 10 15
   Ala Xaa
<210> 33
   <211> 18
   <212> PRT
   <213> Artificial Sequence
<220>
   <223> Synthetic
<220>
   <221> MISC_FEATURE
   <222> (6)..(6)
   <223> Xaa is a tryptophan residue or an oxidant resistant residue
<220>
   <221> MISC_FEATURE
   <222> (18)..(18)
   <223> Xaa is a tryptophan residue or an oxidant resistant residue
<400> 33
   Glu Phe Leu Lys Ala Xaa Tyr Glu Lys Val Ala Glu Lys Leu Lys Glu
   1 5 10 15
   Ala Xaa
<210> 34
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (6)..(6)
   <223> Xaa is a tryptophan residue or an oxidant resistant residue
<220>
   <221> MISC_FEATURE
   <222> (17)..(18)
   <223> Xaa is a tryptophan residue or an oxidant resistant residue
<400> 34
   Asp Phe Xaa Lys Ala Xaa Tyr Asp Lys Val Ala Glu Lys Leu Lys Glu
   1               5                   10                  15
   Xaa Xaa
<210> 35
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (6)..(6)
   <223> Xaa is a tryptophan residue or an oxidant resistant residue
<220>
   <221> MISC_FEATURE
   <222> (17)..(18)
   <223> Xaa is a tryptophan residue or an oxidant resistant residue
<400> 35
   Glu Phe Xaa Lys Ala Xaa Tyr Glu Lys Val Ala Glu Lys Leu Lys Glu
   1 5 10 15
   Xaa Xaa
<210> 36
   <211> 18
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<400> 36
   Asp Lys Leu Lys Ala Phe Tyr Asp Lys Val Phe Glu Xaa Ala Lys Glu
   1 5 10 15
   Ala Phe
<210> 37
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 37
   Asp Lys Xaa Lys Ala Val Tyr Asp Lys Phe Ala Glu Ala Phe Lys Glu
   1 5 10 15
   Phe Leu
<210> 38
   <211> 18
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<400> 38
   Glu Lys Leu Lys Ala Phe Tyr Glu Lys Val Phe Glu Xaa Ala Lys Glu
   1               5                   10                  15
   Ala Phe
<210> 39
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 39
   Glu Lys Xaa Lys Ala Val Tyr Glu Lys Phe Ala Glu Ala Phe Lys Glu
   1               5                   10                  15
   Phe Leu
<210> 40
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 40
   Asp Xaa Leu Lys Ala Phe Val Asp Lys Phe Ala Glu Lys Phe Lys Glu
   1               5                   10                  15
   Ala Tyr
<210> 41
   <211> 18
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<400> 41
   Glu Lys Xaa Lys Ala Val Tyr Glu Lys Phe Ala Glu Ala Phe Lys Glu
   1               5                   10                  15
   Phe Leu
<210> 42
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 42
   Asp Xaa Leu Lys Ala Phe Val Tyr Asp Lys Val Phe Lys Leu Lys Glu
   1               5                   10                  15
   Phe Phe
<210> 43
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 43
   Glu Xaa Leu Lys Ala Phe Val Tyr Glu Lys Val Phe Lys Leu Lys Glu
   1               5                   10                  15
   Phe Phe
<210> 44
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 44
   Asp Xaa Leu Arg Ala Phe Tyr Asp Lys Val Ala Glu Lys Leu Lys Glu
   1               5                   10                  15
   Ala Phe
<210> 45
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 45
   Glu Xaa Leu Arg Ala Phe Tyr Glu Lys Val Ala Glu Lys Leu Lys Glu
   1               5                   10                  15
   Ala Phe
<210> 46
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 46
   Asp Xaa Leu Lys Ala Phe Tyr Asp Arg Val Ala Glu Lys Leu Lys Glu
   1 5 10 15
   Ala Phe
<210> 47
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 47
   Glu Xaa Leu Lys Ala Phe Tyr Glu Arg Val Ala Glu Lys Leu Lys Glu
   1 5 10 15
   Ala Phe
<210> 48
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 48
   Asp Xaa Leu Lys Ala Phe Tyr Asp Lys Val Ala Glu Arg Leu Lys Glu
   1 5 10 15
   Ala Phe
<210> 49
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 49
   Glu Xaa Leu Lys Ala Phe Tyr Glu Lys Val Ala Glu Arg Leu Lys Glu
   1               5                   10                  15
   Ala Phe
<210> 50
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 50
   Asp Xaa Leu Lys Ala Phe Tyr Asp Lys Val Ala Glu Lys Leu Arg Glu
   1               5                   10                  15
   Ala Phe
<210> 51
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 51
   Glu Xaa Leu Lys Ala Phe Tyr Glu Lys Val Ala Glu Lys Leu Arg Glu
   1               5                   10                  15
   Ala Phe
<210> 52
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 52
   Asp Xaa Leu Lys Ala Phe Tyr Asp Arg Val Ala Glu Arg Leu Lys Glu
   1               5                   10                  15
   Ala Phe
<210> 53
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 53
   Glu Xaa Leu Lys Ala Phe Tyr Glu Arg Val Ala Glu Arg Leu Lys Glu
   1               5                   10                  15
   Ala Phe
<210> 54
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 54
   Asp Xaa Leu Arg Ala Phe Tyr Asp Lys Val Ala Glu Lys Leu Arg Glu
   1               5                   10                  15
   Ala Phe
<210> 55
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 55
   Glu Xaa Leu Arg Ala Phe Tyr Glu Lys Val Ala Glu Lys Leu Arg Glu
   1               5                   10                  15
   Ala Phe
<210> 56
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 56
   Asp Xaa Leu Arg Ala Phe Tyr Asp Arg Val Ala Glu Lys Leu Lys Glu
   1               5                   10                  15
   Ala Phe
<210> 57
   <211> 17
   <212> PRT
   <213> Artificial Sequence
<220>
   <223> Synthetic
<220>
   <221> MISC_FEATURE
   <222> (1)..(1)
   <223> Xaa is a tryptophan residue or an oxidant resistant residue
<400> 57
   Xaa Leu Arg Ala Phe Tyr Glu Arg Val Ala Glu Lys Leu Lys Glu Ala
   1 5 10 15
   Phe
<210> 58
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 58
   Asp Xaa Leu Lys Ala Phe Tyr Asp Lys Val Ala Glu Arg Leu Arg Glu
   1 5 10 15
   Ala Phe
<210> 59
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 59
   Glu Xaa Leu Lys Ala Phe Tyr Glu Lys Val Ala Glu Arg Leu Arg Glu
   1 5 10 15
   Ala Phe
<210> 60
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 60
   Asp Xaa Leu Arg Ala Phe Tyr Asp Lys Val Ala Glu Arg Leu Lys Glu
   1               5                   10                  15
   Ala Phe
<210> 61
   <211> 18
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<400> 61
   Glu Xaa Leu Arg Ala Phe Tyr Glu Lys Val Ala Glu Arg Leu Lys Glu
   1               5                   10                  15
   Ala Phe
<210> 62
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 62
   Asp Xaa Leu Lys Ala Phe Tyr Asp Lys Val Ala Glu Lys Leu Lys Glu
   1 5 10 15
   Ala Phe Pro Asp Xaa Leu Lys Ala Phe Tyr Asp Lys Val Ala Glu Lys
   20 25 30
   Leu Lys Glu Ala Phe
   35
<210> 63
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 63
   Asp Xaa Leu Lys Ala Phe Tyr Asp Lys Val Ala Glu Lys Leu Lys Glu
   1 5 10 15
   Phe Phe Pro Asp Xaa Leu Lys Ala Phe Tyr Asp Lys Val Ala Glu Lys
   20 25 30
   Leu Lys Glu Phe Phe
   35
<210> 64
   <211> 37
   <212> PRT
   <213> Artificial Sequence
<220>
   <223> Synthetic
<220>
   <221> MISC_FEATURE
   <222> (2)..(2)
   <223> Xaa is a tryptophan residue or an oxidant resistant residue
<220>
   <221> MISC_FEATURE
   <222> (21)..(21)
   <223> Xaa is a tryptophan residue or an oxidant resistant residue
<400> 64
   Asp Xaa Phe Lys Ala Phe Tyr Asp Lys Val Ala Glu Lys Leu Lys Glu
   1               5                   10                  15
   Ala Phe Pro Asp Xaa Phe Lys Ala Phe Tyr Asp Lys Val Ala Glu Lys
               20                  25                  30
   Leu Lys Glu Ala Phe
           35
<210> 65
   <211> 37
   <212> PRT
   <213> Artificial Sequence
<220>
   <223> Synthetic
<220>
   <221> MISC_FEATURE
   <222> (13)..(13)
   <223> Xaa is a tryptophan residue or an oxidant resistant residue
<220>
   <221> MISC_FEATURE
   <222> (32)..(32)
   <223> Xaa is a tryptophan residue or an oxidant resistant residue
<400> 65
   Asp Lys Leu Lys Ala Phe Tyr Asp Lys Val Phe Glu Xaa Ala Lys Glu
   1               5                   10                  15
   Ala Phe Pro Asp Lys Leu Lys Ala Phe Tyr Asp Lys Val Phe Glu Xaa
               20                  25                  30
   Leu Lys Glu Ala Phe
           35
<210> 66
   <211> 37
   <212> PRT
   <213> Artificial Sequence
<220>
   <223> Synthetic
<220>
   <221> MISC_FEATURE
   <222> (3)..(3)
   <223> Xaa is a tryptophan residue or an oxidant resistant residue
<220>
   <221> MISC_FEATURE
   <222> (22)..(22)
   <223> Xaa is a tryptophan residue or an oxidant resistant residue
<400> 66
   Asp Lys Xaa Lys Ala Val Tyr Asp Lys Phe Ala Glu Ala Phe Lys Glu
   1               5                   10                  15
   Phe Leu Pro Asp Lys Xaa Lys Ala Val Tyr Asp Lys Phe Ala Glu Ala
               20                  25                  30
   Phe Lys Glu Phe Leu
           35
<210> 67
   <211> 37
   <212> PRT
   <213> Artificial Sequence
<220>
   <223> Synthetic
<220>
   <221> MISC_FEATURE
   <222> (2) .. (2)
   <223> Xaa is a tryptophan residue or an oxidant resistant residue
<220>
   <221> MISC_FEATURE
   <222> (21) .. (21)
   <223> Xaa is a tryptophan residue or an oxidant resistant residue
<400> 67
   Asp Xaa Phe Lys Ala Phe Tyr Asp Lys Val Ala Glu Lys Phe Lys Glu
   1               5                   10                  15
   Ala Phe Pro Asp Xaa Phe Lys Ala Phe Tyr Asp Lys Val Ala Glu Lys
               20                  25                  30
   Phe Lys Glu Ala Phe
           35
<210> 68
   <211> 37
   <212> PRT
   <213> Artificial Sequence
<220>
   <223> Synthetic
<220>
   <221> MISC_FEATURE
   <222> (2) .. (2)
   <223> Xaa is a tryptophan residue or an oxidant resistant residue
<220>
   <221> MISC_FEATURE
   <222> (21) .. (21)
   <223> Xaa is a tryptophan residue or an oxidant resistant residue
<400> 68
   Asp Xaa Leu Lys Ala Phe Val Tyr Asp Lys Val Phe Lys Leu Lys Glu
   1               5                   10                  15
   Phe Phe Pro Asp Xaa Leu Lys Ala Phe Val Tyr Asp Lys Val Phe Lys
               20                  25                  30
   Leu Lys Glu Phe Phe
           35
<210> 69
   <211> 37
   <212> PRT
   <213> Artificial Sequence
<220>
   <223> Synthetic
<220>
   <221> **MISC_FEATURE**
   <222> (2) .. (2)
   <223> Xaa is a tryptophan residue or an oxidant resistant residue
<220>
   <221> **MISC_FEATURE**
   <222> (21) .. (21)
   <223> Xaa is a tryptophan residue or an oxidant resistant residue
<400> 69
   Asp Xaa Leu Lys Ala Phe Tyr Asp Lys Phe Ala Glu Lys Phe Lys Glu
   1               5                   10                  15
   Phe Phe Pro Asp Xaa Leu Lys Ala Phe Tyr Asp Lys Phe Ala Glu Lys
               20                  25                  30
   Phe Lys Glu Phe Phe
           35